# EUROPEAN PATENT SPECIFICATION

(11) **EP 2 098 175 B1**
(45) Date of publication and mention of the grant of the patent: **17.08.2011**
(21) Application number: 09003038.8
(22) Date of filing: 03.03.2009
(51) Int. Cl.: A61B 17/122, A61B 17/128

(54) **Clipping device**
Schneidvorrichtung
Dispositif de détourage

(30) Priority: 04.03.2008 JP 2008053505; 06.03.2008 JP 2008056351; 24.03.2008 JP 2008075557; 24.03.2008 JP 2008076045
(43) Date of publication of application: 09.09.2009
(73) Proprietor: FUJIFILM Corporation, Minato-ku Tokyo (JP)
(72) Inventor: Cui, Shengfu, Kanagawa (JP)
(74) Representative: Klunker . Schmitt-Nilson . Hirsch

(56) References cited:
- EP-A- 1 884 209
- US-A1- 2006 224 165

## Description

### BACKGROUND OF THE INVENTION

The present invention relates to an endoscopic clipping device according to the preamble of claim 1 such device is used to effect stopping of bleeding, closing of a puncture, etc. in a living body or the like.

In an endoscopic clipping device, a clip is caused to protrude from a distal end of an endoscope inserted into a living body, and a portion to.be ligated, such as a bleeding portion or a portion from which a lesioned tissue has been removed is pinched by the clip, thereby stopping the bleeding or closing the puncture. A conventional clipping device has a clip whose distal end is open, and a clamping part for closing the clip to effect fastening, with the clip being closed by causing the clamping part to advance with respect to the open clip.

For example, JP 2002-272751 A discloses a construction in which a clip clamping ring is attached to a rear end portion of a clip and in which the clip is pulled with respect to the clip clamping ring to thereby draw the clip into the clip clamping ring, thereby closing a pinching portion at the distal end portion of the clip and grasping living tissues. In the clip clamping ring of JP 2002-272751 A, when the clip is pushed out of an introducing tube (sheath), the clip clamping ring is pushed out together with the clip. This clip clamping ring has two blades that can protrude and retract. When a distal end tip attached to the distal end of the introducing tube is passed by, the blades protrude, and if the clip is pulled thereafter, the clip clamping ring does not retract into the introducing tube. When the clip is pulled in this state, the clip is clamped by the clip clamping ring. After that, the clip clamping ring is allowed to stay in the living body cavity together with the clip.

JP 2006-187391 A discloses a construction in which a clamping ring is fitted onto a rear side portion of a clip and in which the clip is pulled with respect to this clamping ring, whereby the clamping ring is moved to a front side portion of the clip, thereby closing the clip. The clamping ring of JP 2006-187391 A is a truncated-cone-shaped component whose outer diameter increases from the front end toward the rear end. The outer diameter of the rear end of the same is larger than the inner diameter of a tubular front tip mounted to the front end of a sheath. A front side portion of the front tip has an axial slit, and can undergo elastic deformation so as to enlarge the inner diameter. Thus, the clamping ring, which can be pushed out from the front tip toward the front end, cannot be brought back to the former position once it has been pushed out. At the time of clipping, the clamping ring is pushed out of the front tip, and is prevented from retreating by the front tip. In this state, a manipulating wire is pulled to draw the clip into the sheath, thereby effecting clipping.

The clip of JP 2002-272751 A is made of a thin metal strip, and the clip clamping ring is made of a resin, metal, or the like. However, when the clip clamping ring is made of a resin, it may be impossible to obtain a clamping force strong enough to maintain the grasping force of the clip depending upon the size and hardness of the living tissues to be grasped. When the clip clamping ring is made of metal, it is impossible to endow the blade portions with sufficient elasticity, and hence there is a fear of the blades not spreading properly when the front tip is passed by.

In the construction disclosed in JP 2006-187391 A, it is necessary to separately provide, at the front end of the sheath, the front tip of a tapered configuration having a slit, i.e., a separate member of a special configuration. Further, this front tip requires attachment/detachment each time the clip is put into the sheath. JP 2006-187391 A gives no description regarding 3

the material of the clip and the clamping ring. It should be noted, however, that when the clamping ring is made of a resin or the like, it may be impossible to obtain a claming force strong enough to maintain the grasping force of the clip depending upon the size and hardness of the living tissues to be grasped.

EP 1 884 209 A1 discloses a single clipping device in which a head portion attached to the distal end of a manipulating wire can be attached to a retention ring in which a clip having two opposed arm portions is received. The manipulating wire is pushed through a tube or a sheath while the arm portions are received within the retention portion in a collapsed state. When the retaining portion leaves the sheath, two opposed skirt portions extend outwardly so that they abut against the front end of the sheath when the manipulating wire is retracted. The arms of the clip can be closed by retracting the wire. The retraction of the wire also disconnects the retention portion from the manipulating wire.

US 2006/0224165 A1 discloses a clipping device of a magazine type according to the preamble of claim 1. A tube or sheath accommodates a plurality of units, each unit comprising a clip having two arms and a retaining portion which surrounds the middle part of the arms. In a specific embodiment, the clip is formed of a flat metal sheet formed so as to have a pair of claws at the one end and a turned portion at the other end. Therefore, if the turned portion of a preceding clamp is engaged with a successive clamp by means of the claws thereof, there would result a situation in which the orientations of the clips change alternately by 90 degrees.

### SUMMARY OF THE INVENTION

It is an object of the present invention to solve the above-mentioned problems in the related art and to provide a clipping device including a clamping ring realizing both the function of preventing the clip from retreating into the sheath at the time of clipping manipulation and a strong clamping force of the clip, and making it possible to perform clipping and clip loading through easy manipulation.

A clipping device according to the invention comprises the features of claim 1.

### BRIEF DESCRIPTION OF THE DRAWINGS

In the accompanying drawings:
FIGS. 1A and 1B are side sectional view and a front sectional view, respectively, illustrating a clipping device according to Embodiment 1 of the present invention;
FIG. 2 is a perspective view of a clip used in Embodiment 1;
FIGS. 3A through 3C are a front view, a front sectional view, and a bottom view of a connection ring used in Embodiment 1;
FIGS. 4A and 4B are partial sectional views illustrating how the clip is connected and retained by the connection ring;
FIGS. 5A and 5B are a partial plan sectional view and a partial front sectional view schematically illustrating the construction of a manipulating portion;
FIGS. 6A through 6E are sectional views illustrating stepwise the state of the clipping device according to Embodiment 1 during clipping manipulation;
FIGS. 7A and 7B are a front view and a front sectional view of a connection clip package, and FIG. 7c is a sectional view thereof taken along a plane orthogonal to the axis of a case thereof;
FIG. 8 is a partial enlarged view of FIG. 7B;
FIGS. 9A through 9C are partial sectional views illustrating stepwise how clip member loading manipulation is performed from the connection clip package to the sheath;
FIG. 10 is a perspective view of a connecting portion between a connecting member and a manipulating wire;
FIG. 11 is a sectional view of a connection ring used in Embodiment 2;
FIGS. 12A through 12D are partial sectional views of a clipping device according to Embodiment 2;
FIGS. 13A through 13C are a front view, a front sectional view, and a bottom view of a connection ring used in Embodiment 3;
FIGS. 14A and 14B are a front view and a front sectional view of a connection clip package according to Embodiment 4, and FIG. 14C is a sectional view thereof taken along a plane orthogonal to the axis of the case thereof;
FIGS. 15A through 15C are partial sectional views illustrating stepwise how clip member loading manipulation is performed in Embodiment 5;
FIGS. 16 and 17 are a perspective view and a sectional view of a manipulating portion used in Embodiment 6;
FIG. 18 is a perspective view of the manipulating portion according to Embodiment 6 with a slider guide removed therefrom;
FIG. 19A is a perspective view of a guide portion of the slider guide;
FIG. 19B is a schematic developed view of the slider guide;
FIG. 20 is a perspective view of a rotating position regulating member; and
FIG. 21 is a schematic developed view of the slider guide illustrating how clipping manipulation is performed in Embodiment 6.

### DETAILED DESCRIPTION OF THE PREFERRED EMBODIMENTS

In the following, preferred embodiments of the clipping device of the present invention are described with reference to the accompanying drawings.

### Embodiment 1

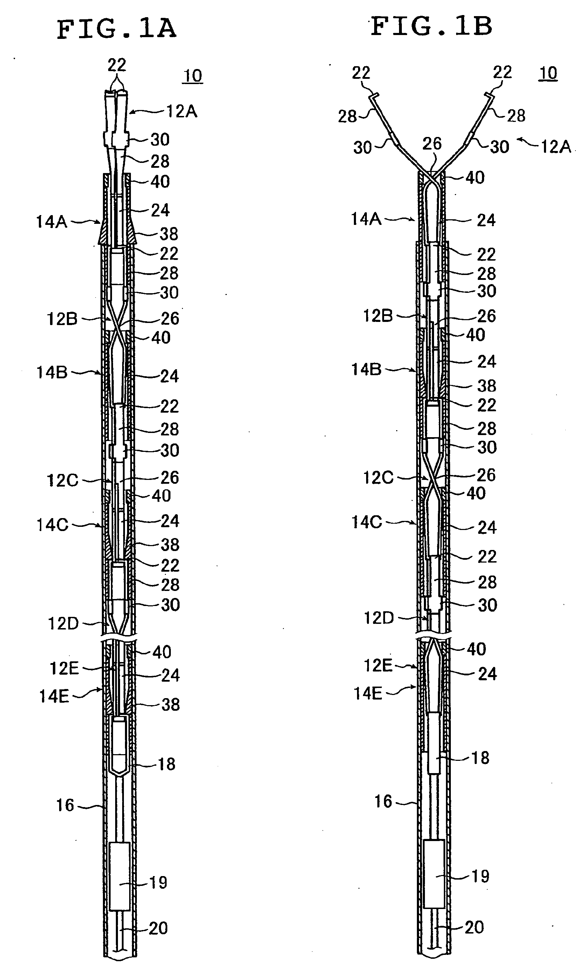

FIGS. 1A and 1B are sectional views of a clipping device 10 according to Embodiment 1 of the present invention, and FIG. 1B is a diagram as seen from an angle differing from FIG. 1A by 90 degrees.

The clipping device 10 is a magazine type clipping device in which clips can be used in succession and which has a plurality of clips 12 (12A, 12B, 12C, 12D, and 12E), a dummy clip 18 connected to the rearmost clip 12D, a manipulating wire 20 connected to the dummy clip via a connecting member 19, and connection rings 14 (14A, 14B, 14C, 14D, and 14E) covering the engagement portions of the adjacent clips 12 to maintain the clips 12 in the connected state, with those components being fitted into a sheath 16. FIGS. 1A and 1B illustrate an initial state immediately before the start of clipping manipulation by the foremost clip 12.

One clip 12 and one connection ring 14 corresponding to the clip 12 form one endoscopic bleeding stop clip member, and the clipping device 10 includes a plurality of such bleeding stop clip members loaded into the interior of the distal end portion of the elongated sheath 16. The terminal end of the successive bleeding stop members is engaged with the dummy clip 18, and the manipulating wire 20 extends to the proximal end of the sheath 16 to be connected to a manipulating portion described below. When the manipulating wire 20 is drawn out by a predetermined length from the manipulating portion, and the dummy clip 18 is moved in one direction by the predetermined length, the series of clips 12 move by the same amount, and the foremost clip 12 is clamped by the connection ring 14 retaining the same, whereby clipping for stopping bleeding, marking, etc. is effected by the foremost clip 12. When the clipping by the foremost clip 12 has been completed, the sheath 16 is pulled toward the manipulating portion side by a predetermined length, whereby the next clip 12 is placed in a usable state (standby state), thus making it possible to perform clipping successively.

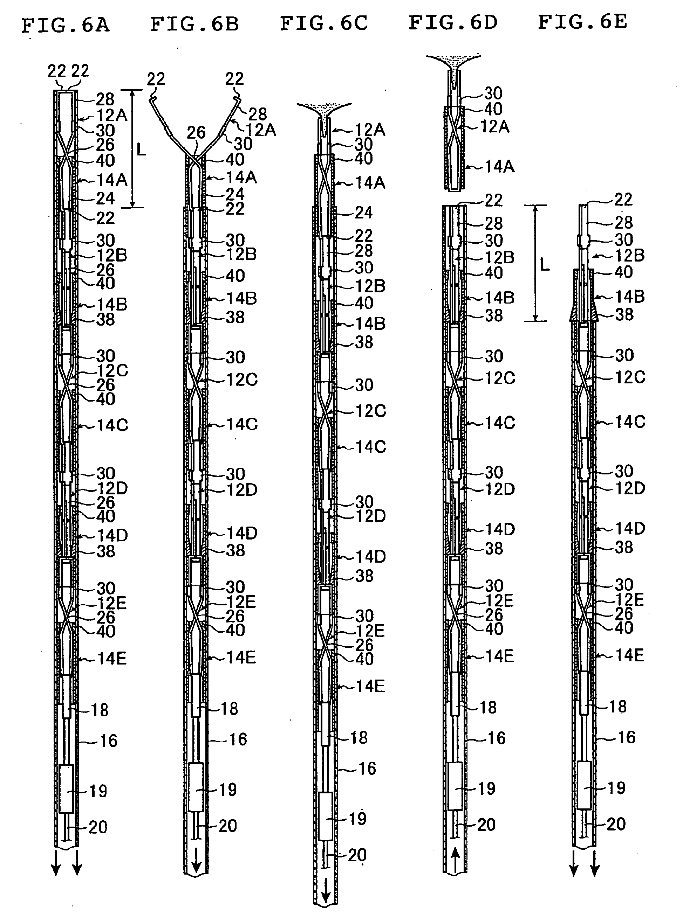

While in FIGS. 1A and 1B the foremost clip 12A protrudes from the distal end of the sheath 16, when loading the clips 12, etc. into the sheath 16, setting is effected such that the foremost clip 12A is completely accommodated within the sheath 16 as illustrated in FIG. 6A. Further, while in FIGS. 1A and 1B the number of clips 12 is five, that is, the clipping device is of a five-shooter type, it is possible for the clips 12 to be provided in any number not less than two.

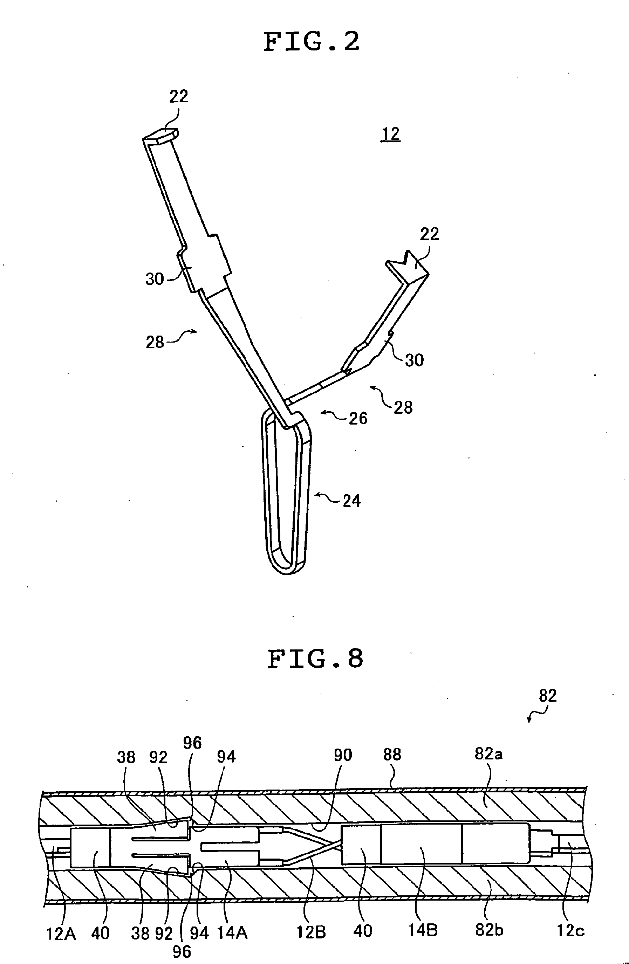

FIG. 2 is a perspective view of the clip 12. The clip 12 is a closed clip having a turned portion 24 turned by 180 degrees with respect to claw portions 22. That is, in forming the clip 12, a single elongated plate is bent by 180 degrees to form a closed end, and then both ends thereof are caused to cross each other. Further, the end portions are bent so as to be opposed to each other, thereby forming the claw portions 22 to two open ends. On the open-end side of the crossing portion 26, there exist arm portions 28, and, on the closed-end portion thereof, there exists the turned portion 24. At the central portion of each arm portion 28, there is formed a partially widened projection 30. The clip 12 may be formed of a metal with biocompatibility. For example, it is possible to use SUS 631, which is a spring stainless steel.

In the clip 12, the forward end portion (a clamping portion 40 described below) of the connection ring 14 fitted onto the crossing portion 26 moves by a predetermined amount toward the claw portions 22 while pressurizing the arm portions 28, whereby the arm portions 28 and the claw portions 22 are closed, with the claw portions 22 exerting a predetermined fit-engagement force.

To reliably grasp an object, the claw portions 22 are formed as V-shaped male type and female type ones. Further, as illustrated in FIG. 2, the arm portions 28 of the clips 12 gradually increase in width from the crossing portion 26 toward the projections 30.

The projections 30 have a width larger than that of the portions of the distal end side opening and the proximal end side opening of the connection ring 14 abutted by the projections 30. Thus, while the portions of the clip 12 other than the projections 30 can enter the interior of the connection ring 14, the projections 30 cannot enter the interior either from the distal end side or the proximal end side of the connection ring 14.

It is only necessary for the projections 30 to be wide enough to prevent intrusion into the interior of the connection ring 14. However, as described below, the distal end side end portions of the protrusions 30 (upper end portions as seen in FIG. 2) abut the proximal end of the connection ring 14 retaining the arm portions 28 of the clip 12, and serve to place the connection ring 14 in position with respect to the clip 12, and hence it is desirable for the distal end side end portions of the projections 30 to be substantially perpendicular with respect to the axial direction of the clip 12, making it possible to reliably secure the portions held in contact with the proximal end of the connection ring 14.

When, for example, performing stamping on a strip plate constituting the material of the clip 12, the projections 30 are provided beforehand, and the plate obtained by stamping is worked as described above, thereby forming the projections 30. The plate thickness at the projections 30 may be different from the thickness of the other portions of the arm portions 28. Further, it is also possible to fold the end portions in the width direction (horizontal direction) thereof inwardly in the diverging direction of the clip 12. In this case, it is possible to enhance the strength of the projections 30.

The positions of the projections 30 of the arm portions 28 are determined as follows.

Regarding the second clip 12 onward, the distal end side portions of the projections 30 having a width not allowing them to enter the connection ring 14 (hereinafter referred to as upper end portions) are situated so as to abut the proximal end of the connection ring 14 retaining the arm portions 28 of each clip 12 when the clipping device is placed in the state as illustrated in FIGS. 1A and 1B, that is, in the initial state immediately before the start of the clipping manipulation by the foremost clip 12A, in which the plurality of clips 12 have been connected together and loaded into the sheath 16.

In the clipping device 10, to cause the foremost clip 12 to protrude from the sheath 16, the sheath 16 is retracted with respect to the clips 12. At this time, due to the frictional force between the sheath 16 and the connection rings 14 fitted into the sheath 16, there is exerted on the connection rings 14 a force that would cause them to retreat with respect to the clips 12 together with the sheath 16. However, by keeping the upper end portions of the projections 30 of the clips 12 in contact with the proximal ends of the connection rings 14, the connection rings 14 are prevented from moving even when retracting the sheath 16, making it possible to maintain the clips 12 and the connection rings 14 in the initial positional relationship.

The proximal end side portions of the projections 30 having a width not allowing them to enter the connection rings 14 are at the movement positions at the distal ends of the connection rings 14 or slightly on the distal end side thereof to obtain a pre-set fit-engagement force at the claw portions 22 of the clips 12 through the movement of the connection rings 14 at the time of clamping of the clips 12 by the connection rings 14.

The distal ends of the connection rings 14 (i.e., clamping portions 40 thereof) move to the positions where they abut the projections 30 of the clips 12 or to positions immediately below the same, whereby the clips 12 can exert a predetermined fit-engagement force, e.g., the maximum fit-engagement force, at the claw portions 22.

Further, by providing the clip 12 with the projections 30, it is possible to prevent the connection rings 14 from moving toward the distal end side by an amount larger than the predetermined amount and to avoid excessive clamping of the clip 12 and, conversely, loosening of the clamping of the clip 12.

As illustrated in FIGS. 1A and 1B, the claw portions 22 of the second clip 12B are engaged with the turned portion 24 of the first clip 12A and retained by the connection ring 14A in a closed state, whereby the first clip 12A and the second clip 12B are connected together. As illustrated in FIG. 1A, the claw portions 22 of the second clip 12B are engaged with the turned portion 24 of the first clip 12A in a direction orthogonal thereto, with the first clip 12A and the second clip 12B being differing in orientation by 90 degrees. Similarly, the clips 12C, 12D, and 12E are connected together, with their orientations alternately differing by 90 degrees.

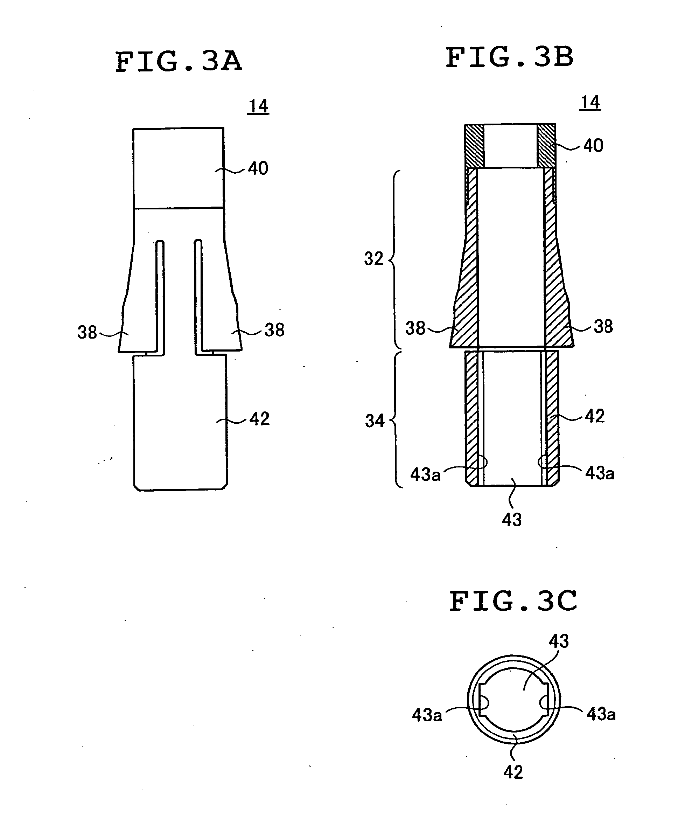

Each ring 14 is fitted into the sheath 16 so as to be capable of advancing and retreating while covering the engagement portion between two clips 12 and maintaining their connected state. That is, the outer diameter of the connection rings 14 is substantially the same as the inner diameter of the sheath 16 so that they can smoothly advance and retreat within the sheath 16 as the clips 12 move. FIGS. 3A through 3C schematically illustrate the construction of each connection ring 14. FIG. 3A is a front view of the connection ring 14, FIG. 3B is a sectional view thereof, and FIG. 3C is a bottom view thereof.

The connection ring 14 includes a clamping portion 40 and a retaining portion 42. In the connection ring 14, the clamping portion 40 formed of metal is fixed to the forward end of the retaining portion 42 formed of resin, and the two members form an integral structure. The retaining portion 42 formed of resin serves to maintain the connected state and to retain the clip within the connection ring, and the clamping portion 40 formed of metal serves to clamp the clip. It is also possible for the connection ring 14 to be formed by a single member if it can provide the functions of both the clamping portion 40 and the retaining portion 42.

The clamping portion 40 is a cylindrical (ring-like) metal component mounted to the forward end side of the connection ring 14, and has a hole whose inner diameter is larger than the width of the portion of the clip 12 in the vicinity of the crossing portion 26 and smaller than the width of the projections 30. Thus, while the clamping portion 40 can move in the vicinity of the crossing portion 26 of the clip 12 it retains, it cannot be detached to the forward end side beyond the projections 30. That is, the projections 30 function as a stopper determining the movement limit of the connection ring 14 advancing with respect to the clip 12.

The clamping portion 40 is set at a predetermined position in the vicinity of the crossing portion 26 of the clip 12. The clamping portion 40 moves from its initial position, i.e., from the crossing portion 26 toward the projections 30, with the arm portions 28 of the clip 12 increasing in width, whereby it closes the arm portions 28 of the diverging clip 12 to effect fixation and clamping. As the material of the clamping portion 40, there is used a metal with biocompatibility, for example, a stainless steel SUS 304. By forming the clamping portion 40 of metal, it is possible to exert on the metal clip 12 a frictional force, which serves as the clamping force.

The retaining portion 42 is a schematically cylindrical (ring-like) component formed by resin molding. The retaining portion 42 has a first region 32 retaining the preceding clip 12 and a second region 34 which is a connection retaining region retaining the next clip 12 while connected to the preceding clip.

The first region 32 has a large circular hole capable of accommodating the turned portion 24 of the clip 12 and larger than the hole of the clamping portion 40. On the outer surface of the forward end portion of the first region 32, there is formed a stepped portion onto which the clamping portion 40 is to be fitted, and the clamping portion 40 and the retaining portion 42 are fit-engaged with each other through close fit such that they are not detached from each other while loaded in the sheath 16 and during clipping manipulation. Further, the first region 32 has skirt portions 38 each diverging while inclined in a skirt-like fashion with respect to the axis of the connection ring 14 main body.

The forward end side, that is, the upper, base portion of the skirt portion 38 as seen in FIGS. 3A and 3B is connected to the main body of the retaining portion 42, whereas the lower, diverging portion thereof is partially separated from the main body to be radially diverged or closed. Two skirt portions 38 are formed so as to be separated from each other by 180 degrees at the same position in the pulling direction for the clip 12, that is, in the vertical direction in FIGS. 3A and 3B.

When left as they are, that is, when in a state in which no external force is being imparted thereto, the skirt portions 38 are diverged in a skirt-like fashion as illustrated in fig. 3A. At this time, the interior of the first region 32 of the retaining portion 42 forms a columnar space as illustrated in FIG. 3B. When loading the connection rings 14 into the sheath 16, the following takes place: in the case, for example, of the second connection ring 14B illustrated in FIG. 1B, the skirt portions 38 are pushed in to enter the internal space, and the inner peripheral side portions of the skirt portions 38 pressurize the side surface (edge portion) of the turned portion 24 of the clip 12B retained by the first region 32, thus retaining the clip 12B such that it does not move in the rotating direction and the advancing/retreating direction within the connection ring 14B. It is also possible for the skirt portions 38 to pressurize and retain the clip retained by the second region 34, that is, the succeeding clip.

As in the case of the first connection ring 14A illustrated in FIG. 1A, the skirt portions 38 extend beyond the forward end of the sheath 16, and are opened due to their own elasticity, releasing the retention of the clip 12A and becoming wider than the inner diameter of the sheath 16 to prevent the connection ring 14A from retracting into the sheath 16. In this state, the manipulating wire 20 is pulled, and the clip 12A retreats, whereby the connection ring 14A advances relative to the clip 12A to clamp the clip 12A.

Thus, it is necessary for the skirt portions 38 to have elasticity so that they can be closed inwardly within the sheath 16 and widen in a skirt-like fashion when they get out of the forward end of the sheath 16 and released from the external force. At the same time, it is also necessary for the skirt portions 38 to exhibit rigidity enabling them to retain the clip 12 within the sheath 16 and to withstand the repulsive force of the clamping force of the clip 12 at the forward end of the sheath 16.

From the above viewpoints, as the material of the retaining portion 42, there is used a material exhibiting biocompatibility and providing the requisite elasticity and rigidity for the skirt portions 38. As for their configuration, it is determined so as to satisfy the requirements in terms of elasticity and rigidity for the skirt portions 38. As the material of the retaining portion 42, it is possible to use, for example, polyphenylsulfone (PPSU). From the viewpoint of ease of production, it is desirable for the retaining portion 42 to be formed as an integral molding.

The second region 34 is provided on the proximal end side of the first region 32. The succeeding clip 12 engaged with the clip 12 retained by the first region 32 is retained in a state in which the claw portions 22 thereof are closed while holding the closed end (tail portion) of the turned portion 24 of the preceding clip 12 therebetween.

The length of the second region 34 is substantially equal to the movement length required for the clamping portion 40 set at the initial position with respect to the clip 12 to move until the clamping of the clip 12 is completed. That is, while the clip 12 retreats relative to the connection ring 14 to be clamped, the second region 34 of the connection ring 14 maintains the connection between the two clips 12 retained therein, allowing the pulling force of the rear clip 12 to be transmitted to the front clip 12, and when the clamping has been completed, the engagement portion of the two clips 12 is detached from the second region 34, thereby canceling the connection between the clips 12.

As illustrated in FIG. 3C, the second region 34 has a hole 43 having the same inner diameter as the proximal end side portion of the first region 32, and further, two grooves (recesses) 43a opposed to each other are formed. The grooves 43a can accommodate the arm portions 28 of the clip 12 retained in the second region 34, with the claw portions 22 being closed.

The grooves 43a are provided at two positions in the direction in which the claw portions 22 of the clip 12 retained in the second region 34 are opened and closed (horizontal direction in FIGS. 3B and 3C). The plate surfaces of the arm portions 28 of the clip 12 retained in the second region 34 abut the inner walls of the grooves 43a. The width (opening width) of the grooves 43a is slightly larger than the maximum width of the arm portions 28 of the clip 12, and the distance from the wall surface of one groove 43a to the wall surface of the other groove 43a is substantially equal to the sum total of the lengths of the two claw portions 22 of the clip 12 (length in the diverging direction). The width of the grooves 43a is smaller than the width of the projections 30 formed on the arm portions 28. Thus, the projections 30 of the clip 12 retained in the second region 34 cannot enter the grooves 43a.

The distance between the wall surfaces of the two grooves 43a is such that the engagement between the turned portion 24 of the preceding clip 12 and the claw portions 22 of the next clip 12 is not canceled, and the distance is smaller than the sum total of the lengths of the two claw portions 22 and the width of the portion of the turned portion 24 engaged with the claw portions 22.

For example, the claw portions 22 of the clip 12 retained in the second region 34 may slightly overlap each other, or the connection of the clip with the preceding clip 12 may be maintained, with a slight gap being left between the claw portions 22.

The engagement portion between the two clips 12 is retained in the portion of the second region 34 close to the boundary between the second region 34 and the first region 32. Inside the sheath 16, the turned portion 24 of the preceding clip 12 (e.g., the clip 12B in the connection ring 14B illustrated in FIG. 1B) is retained by the closed skirt portions 38 in the first region 32, and hence the advancing/retreating movement and rotating movement of the clip is restrained. The next clip 12 (e.g., the clip 12C in the connection ring 14B illustrated in FIG. 1B) engaged with the preceding clip 12 is retained in an orientation differing by 90 degrees from the preceding clip by the grooves 43a of the second region 34, whereby rotating movement of the clip is restrained, and the clip is engaged with the preceding clip restrained in advancing/retreating movement, thereby restraining the advancing/retreating movement thereof. That is, the engagement portion between the front and rear clips is retained by the connection ring 14 with very little play.

As illustrated in FIGS. 1A and 1B, the claw portions 22 of the second clip 12B are engaged with the turned portion 24 of the first clip 12A, and the engagement portion is retained by the connection ring 14A. The claw portions 22 of the second clip 12B are retained in the closed state by the inner wall of the connection ring 14A (second region 34 thereof). As a result, the connection of the first clip 12A and the second clip 12B is maintained. Similarly, the connection of the second clip 12B and the third clip 12C is maintained by the connection ring 14B, the connection of the third clip 12C and the fourth clip 12D is maintained by the connection ring 14C, the connection of the fourth clip 12D and the fifth clip 12E is maintained by the connection ring 14D, and the connection of the fifth clip 12E and the dummy clip 18 is maintained by the connection ring 14E.

The rearmost clip 12E is engaged with the dummy clip 18, which is not used for clipping. At its forward end portion, the dummy clip 18 has a resilient portion of a configuration similar to that of the open end side half as from the crossing portion of the clip 12. The resilient portion is engaged with the turned portion of the clip 12E, with the claw portions thereof being closed, and releases the clip 12E when the claw portions are opened. At the proximal end portion of the dummy clip 18, there exits the connecting member 19, to which the manipulating wire 20 is connected.

The sheath 16 is formed, for example, of a flexible coil sheath formed through intimate winding of metal wire. The inner diameter of the sheath 16 is one allowing canceling of the engagement between the turned portion 24 of the preceding clip 12 and the claw portions 22 of the next clip 12. That is, the inner diameter of the sheath 16 is larger than the sum total of the lengths of the two claw portions 22 and the width of the turned portion 24 engaged with the claw portions 22.

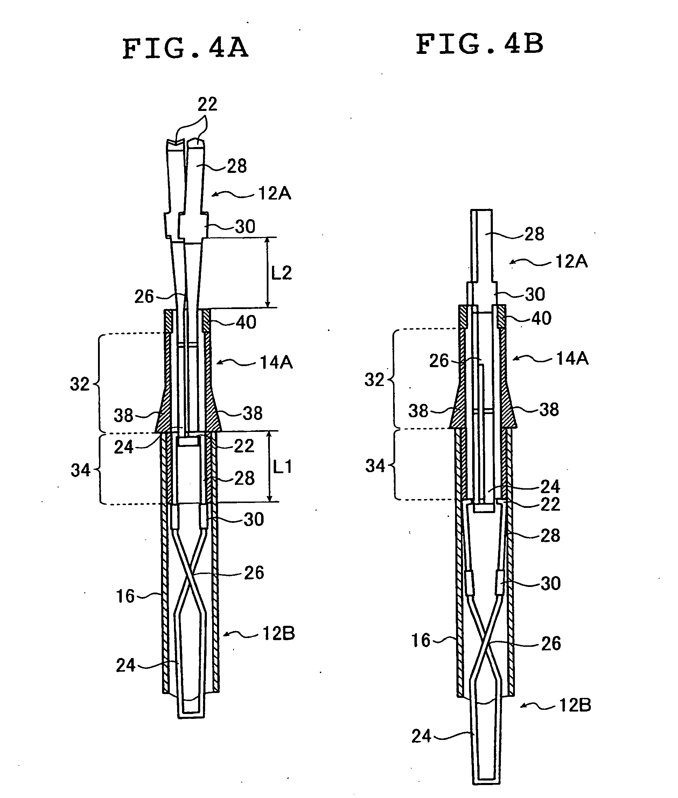

Here, the construction of each clip 12 and each connection ring 14 and the operation of each portion are described in detail taking the connection ring 14A and the clips 12A and 12B retained thereby as an example. FIGS. 4A and 4B are sectional views illustrating stepwise the condition of the clips 12A and 12B and the connection ring 14A during the clipping manipulation of the foremost clip 12A. FIG. 4A corresponds to an enlarged view of the distal end portion of the clipping device of FIG.1A. In FIG. 4A, the connection ring 14B retaining the clip 12B is omitted. Further, the operation described below also applies to the other, succeeding clips 12 and connection rings 14.

In the state illustrated in FIG. 4A, the connection ring 14A is fitted onto the clip 12A and the clip 12B such that the clamping portion 40 thereof is at a predetermined initial position on the clip 12A. This initial position is in the vicinity of the crossing portion 26 of the clip 12A. The clamping portion 40 does not clamp the clip 12A, and the arm portions 28 of the clip 12A are diverged to the maximum.

The turned portion 24 of the clip 12A is accommodated in the first region 32 of the connection ring 14A, and the arm portions 28 of the clip 12B is retained in the second region 34 of the connection ring 14A, with the claw portions 22 being closed with the tail portion of the clip 12A therebetween. The forward end of the clip 12B substantially coincides with the upper end of the second region 34 of the connection ring 14A, and the upper ends of the projections 30 of the clip 12B abut the lower end of the connection ring 14A. Thus, the length L1 as measured from the forward end of the clip 12B to the upper ends of the projections 30 is substantially equal to the length of the second region 34 of the connection ring 14A.

In the state in which, as in the case of the second connection ring onward, i.e., the connection rings 14B through 14D, of FIGS. 1A and 1B are accommodated in the sheath 16, and the skirt portions 38 are closed to retain the clips 12B through 12D in the first regions 32, there is hardly any vertical play in the engagement portions between the front and rear clips 12. Also in FIG. 4A, such a state is substantially maintained.

When the tail portion of the clip 12A and the forward end of the clip 12B are engaged with each other with no play, and the upper ends of the projections 30 of the clip 12B abut the lower end of the connection ring 14A, the clamping portion 40 is brought to a predetermined initial position on the clip 12A. That is, the projections 30 of the clip 12 also serve to determine the initial position of the clamping portion 40.

In the state of FIG. 4A, the length L2 as measured from the lower ends of the projections 30 of the clip 12A to the forward end of the connection ring 14 (clamping portion 40 thereof) is substantially equal to the above-mentioned length L1. The lengths L1 and L2 are equal to the movement amount of the connection ring 14A with respect to the clip 12A to clamp the clip 12A, and are substantially equal to the pulling amount of the manipulating wire 20 (see FIGS. 1A and 1B) causing the clip 12A, etc. to retreat relative to the connection ring 14A, etc.

When, in the state of FIG. 4A, the manipulating wire 20 is pulled by the predetermined amount L2, the clip 12A moves by the length L2 with respect to the connection ring 14A, and, as illustrated in FIG. 4B, the lower ends of the projections 30 of the clip 12A are brought to the position where they abut the forward end of the connection ring 14A or a position immediately above the same.

When the connection ring 14A is brought to a position immediately below the projections 30, the clip 12A exerts a predetermined fit-engagement force at the claw portions 22, e.g., the maximum fit-engagement force thereof, whereby the clamping of the clip 12A by the clamping portion 40 of the connection ring 14 is completed.

By pulling the manipulating wire 20 by the length L2, the clip 12B also moves by the same amount as the clip 12A. That is, the clip 12B moves by the length L1 of the second region 34, which is substantially equal to the above-mentioned length L2, and the forward end of the clip 12B leaves the proximal end of the connection ring 14A, with the engagement portion between the clip 12A and the clip 12B being detached from the second region 34 of the connection ring 14A.

In this way, in the initial state, the clamping portion 40 of the connection ring 14A is set at a fixed initial position of the preceding clip 12A, that is, at a position at the distance L2 from the lower ends of the projections 30 of the clip 12A. By pulling the manipulating wire 20 each time by the fixed pulling amount (stroke) L2, and by moving the clamping portion 40 to the lower ends of the projections 30 of the clip 12A, the clamping of the clips 12 can be completed.

As described above, in the clipping device 10, the sheath 16 is caused to retreat with respect to the clip 12A in order to bring the device from the state in which the foremost clip 12A is completely accommodated in the sheath 16 (state of FIG. 6A described below) to the state in which the foremost clip 12A protrudes from the forward end of the sheath 16 as illustrated in FIG. 4A. If, at this time, the connection ring 14A is allowed to move together with the sheath 16, the position of the connection ring 14A is deviated to the proximal end side with respect to the clip 12A, and the distance from the lower ends of the projections 30 of the clip 12A to the forward end of the connection ring 14A becomes larger than L2. Then, if the manipulating wire 20 is pulled by the predetermined amount L2, the connection ring 14A does not reach the predetermined position on the clip 12A, that is, the lower end of the projections 30, making it impossible to compete the clamping of the clip 12A.

The front and rear clips 12A and 12B are retained by the connection ring 14A in a state in which there is no play in the engagement portion. As a result, by pulling the manipulating wire 20 each time by a fixed pulling amount (stroke) L2 = L1, it is possible to effect the clamping of the clip 12A and the canceling of the connection between the front and rear clips 12A and 12B.

However, if, when causing the sheath 16 to retreat with respect to the clip 12A, the connection ring 14A moves together with the sheath 16, and further, if the clip 12A retained by the skirt portions 38 of the connection ring 14A also moves together with the connection ring 14A, play is generated in the engagement portion of the clips 12A and 12B. Then, if the clip 12B moves toward the proximal end side through pulling of the manipulating wire 20, the clip 12A does not move by the amount corresponding to the play generated, and hence, if the manipulating wire 20 is pulled by the predetermined pulling amount L2, it is impossible to effect the clamping of the clip 12A and the canceling of the connection.

In contrast, in the clipping device 10, the projections 30 of the clip 12B are held in contact with the proximal end of the connection ring 14A, and movement of the connection ring 14A when pulling down the sheath 16 is prevented, whereby it is possible to maintain the mutual positional relationship between the connection ring 14A, the clip 12A, and the clip 12B. Thus, it is possible to always maintain a fixed pulling amount (stroke) for the manipulating wire 20, making it possible to perform a stable, high precision manipulation.

Further, due to the projections 30 of the clip 12B, the mutual positional relationship between the clips 12A and 12B and the connection ring 14A is maintained, whereby it is possible to cause the clip 12A to protrude by a fixed protruding amount from the connection ring 14A in the initial state immediately before the start of the clipping manipulation, making it possible to obtain a predetermined diverging amount for the claw portions 22 of the clip 12A.

In another form, instead of providing the clip 12 with the projections 30, a step may be provided between the first region 32 and the second region 34 in the connection ring 14, and the forward end of the lower clip 12 (bent portions at the forward ends of the arm portions 28) is caused to abut the step portion, thereby preventing rearward movement of the connection ring 14 with respect to the clip 12. For example, at the forward end position of the clip 12B retained by the connection ring 14A, the inner diameter of the first region 32 may be made smaller than the inner diameter of the second region 34, making it impossible for the forward end of the clip 12B retained in the second region 34 to enter the first region 32.

However, in a case in which the connection ring 14 is a very small component whose outer diameter is, for example, 2 mm or less, it is difficult to provide a large step therein, and to provide a sufficient contact portion to be held in contact with the forward end of the clip 12. In such a case, it is more effective to provide the projections 30 on the clip 12.

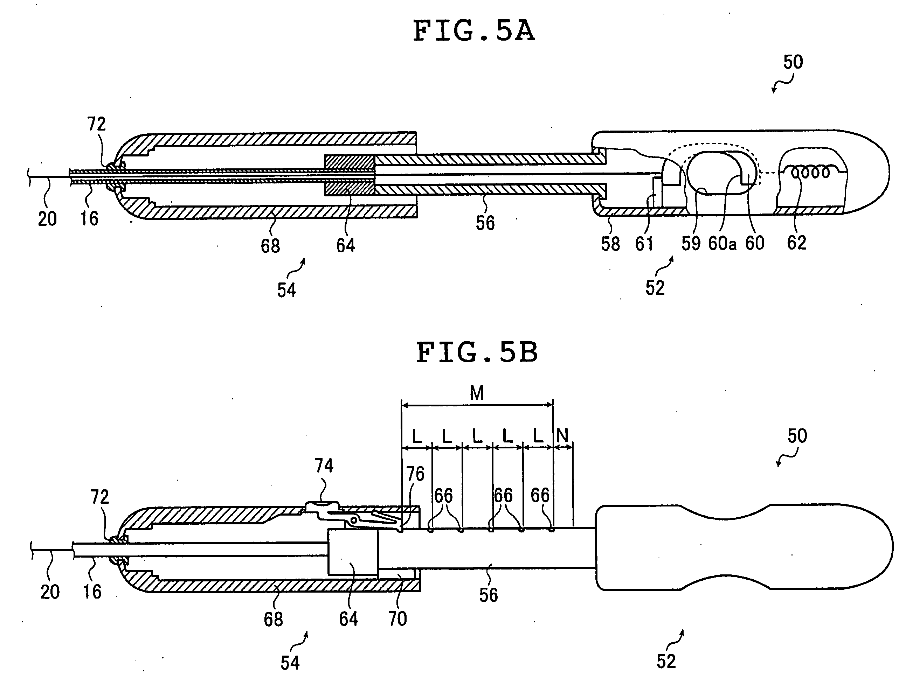

The proximal ends of the manipulating wire 20 and the sheath 16 are attached to the manipulating portion. FIGS. 5A and 5B schematically illustrate an example of the construction of the manipulating portion. In FIGS. 5A and 5B, the left-hand side is the forward end side connected to the clipping device 10, and the right-hand side is the rear end side (or the proximal end side). A manipulating portion 50 includes a wire manipulating handle 52 constituting a manipulating portion main body, and a sheath manipulating handle 54 serving as a grasping portion for grasping the proximal end portion of the sheath, with the sheath manipulating handle 54 being slidable with respect to the wire manipulating handle 52.

The wire manipulating handle 52 includes a cylindrical case 58, a positioning pipe 56 fixed coaxially to the forward end of the case 58, and a lever 60 and a spring 62 retained inside the case 58.

The lever 60 is retained inside the case 58 so as to be movable in the longitudinal direction (axial direction of the wire manipulating handle 52). A rear end part of the lever 60 appears through a window 59 provided at the central portion of the case 58, and the operator can hook his finger onto the rear end part of the lever 60 to pull the lever 60 toward the rear end side. The spring 62 is attached to the rear end of the lever 60. The spring 62 is compressed by pulling the lever 60 rearwards, and when the pulling force on the lever 60 is released, the spring 62 forwardly pushes back the lever 60 by repulsive force. As a result, the lever 60 is restored to the former position (home position).

The rearward movement limit for the lever 60 is determined by the window 59. That is, the position where the surface 60a of the lever 60 onto which the finger is hooked coincides with the rear end of the window 59 is the movement limit for the lever 60. It is also possible to provide a regulating plate at the rear of the lever 60, and to determine the rearward movement limit for the lever 60 through abutment of the rear end of the lever 60 against the regulating plate.

A regulating plate 61 is provided in front of the lever 60 to determine the home position of the lever 60. The lever 60 is urged by the spring 62 and moves forwards until it abuts the regulating plate 61 to return to the home position.

In this way, the lever 60 can move longitudinally by a fixed amount between the home position and the rearward movement limit.

While in FIG. 5A the spring 62 is formed of a coil spring, this should not be construed restrictively. It is only necessary for the spring 62 to be capable of forwardly urging the lever 60. Thus, is also possible to use a plate spring or some other elastic member.

Fixed to the forward end of the lever 60 is the manipulating wire 20 for pulling the clips 12. The manipulating wire 20 extends through the sheath manipulating handle 54 and the positioning pipe 56 to reach the lever 60.

When the operator inserts his finger into the window 59 and pulls the lever 60 to move the lever 60 backwards, the manipulating wire 20 attached to the forward end of the lever 60 also moves, and the forward end of the manipulating wire 20 moves backwards. When the pulling force applied to the lever 60 is canceled and the lever 60 is restored to the former position, the manipulating wire 20 also moves, with its forward end returning to the former position.

The pulling amount of the manipulating wire 20 in the clipping manipulation is a very small amount, e.g., 3.1 mm. Thus, in order to give a reliable operational feel at the manipulating portion 50, a-pulling amount magnifying mechanism for the manipulating wire 20 may be provided between the pulling amount of the manipulating wire 20 and the manipulating amount of the lever 60, making the movement amount of the lever 60 a predetermined number of times the movement amount of the manipulating wire 20.

The positioning pipe 56 is a hollow pipe-like member through which the manipulating wire 20 passes. The inner diameter of the positioning pipe 56 is larger than the outer diameter of the sheath 16, making it possible to insert the sheath 16 into the positioning pipe 56. As illustrated in FIG. 5B, a plurality of notches 66 are formed in the upper surface of the positioning pipe 56 at predetermined axial intervals L. The forward end portion of the positioning pipe 56 is inserted into the sheath manipulating handle 54, and a detachment prevention ring 64 is attached to the forward end portion thereof.

As illustrated in FIG. 5A, at the center of the detachment prevention ring 64, there is formed a hole slightly larger than the outer diameter of the sheath 16. The detachment prevention ring 64 retains the sheath 16 so as to allow the sheath 16 move in the axial direction.

The sheath manipulating handle 54 has a cylindrical case 68, a support block 70, and a sheath retaining ring 72.

The support block 70 is arranged at the rear end of the sheath manipulating handle 54, and slidably supports the positioning pipe 56 inserted into the sheath manipulating handle 54. Further, as illustrated in FIG. 5B, the forward end side surface of the support block 70 abuts the detachment prevention ring 64 attached to the forward end of the positioning pipe 56, preventing the positioning pipe 56 from being detached from the sheath manipulating handle 54.

The sheath retaining ring 72 is provided at the forward end of the case 68 on the axis of the sheath manipulating handle 54, and fixedly retains the outer periphery of the sheath 16 inserted into the sheath manipulating handle 54. Thus, when the sheath manipulating handle 54 moves, the sheath 16 moves together with the same.

The sheath manipulating handle 54 further has a button 74 protruding out of the case 68 and a claw 76 provided inside the case 68 and interlocked with the movement of the button 74. The claw 76 is urged so as to be pressed against the positioning pipe 56, and is caught by the notches 66 of the positioning pipe 56, thereby effecting positioning on the sheath manipulating handle 54 with respect to the wire manipulating handle 52 and stopping the movement thereof.

When the button 74 is depressed, the claw 76 is raised above the notches 66, enabling the wire manipulating handle 52 to move with respect to the sheath manipulating handle 54. When the hand is released from the button 74 and the sheath manipulating handle 54 is moved with respect to the wire manipulating handle 52, its movement is stopped when the claw 76 is caught by the next notch 66. Thus, assuming that the interval L of the notches 66 is one stroke, the one sheath manipulating handle 54 and the sheath 16 can move by the stroke length L. The magnitude of L is, for example, 15.5 mm.

When the sheath 16 moves with the movement of the sheath manipulating handle 54, the proximal end side end portion of the sheath 16 advances through the hole of the detachment prevention ring 64 to enter the interior of the positioning pipe 56.

Next, the operation of the magazine type clipping device 10 is described. FIGS. 6A through 6E are partial sectional views illustrating stepwise the condition of the clipping device 10 during clipping manipulation.

First, as illustrated in FIG. 6A, after five bleeding stop clip units (hereinafter simply referred to as clipping units) formed of the clips 12A through 12E and the connection rings 14A through 14E have been loaded into the sheath 16, the sheath 16 is inserted into the forceps channel of an endoscope. As illustrated in FIG. 6A, in this example, the forward end of the clip 12A is substantially matched with the forward end of the sheath 16.

The foremost clip 12A is retained in the closed state by the inner wall of the sheath 16. Each of the connection rings 14A through 14E is fitted such that the clamping portion 40 thereof is situated in the vicinity of the crossing portion 26 of each of the clips 12A through 12E. At this time, the upper ends of the projections 30 of the clips 12B through 12E are respectively situated directly below the connection rings 14A through 14D.

When the forward end of the sheath 16 reaches the forward end of the insert portion of the endoscope inserted into the living body, and protrudes from the forward end of the endoscope, in the manipulating portion 50 illustrated in FIGS. 5A and 5B, the sheath manipulating handle 54 is pulled such that the claw 76 of the sheath manipulating handle 54 moves by the length L from the first notch 66 to the second notch 66. Since the sheath 16 is fixed to the sheath manipulating handle 54, the sheath 16 retreats by the same amount L as the movement amount L of the sheath manipulating handle 54. Through this manipulation, solely the sheath 16 is drawn to the manipulating portion side, with the manipulating wire 20 remaining stationary.

When the sheath 16 is pulled by the predetermined amount L, which corresponds to the distance between the first notch 66 and the second notch 66, the forward end of the sheath 16 is lowered to a position where the skirt portions 38 of the foremost connection ring 14A are opened, and the claw portions 22 of the clip 12A protruding from the sheath 16 are diverged by the urging force, whereby the state as illustrated in FIG. 6B is attained. As a result, it is possible to use the first clip 12A. In FIG. 6B, the skirt portions 38 of the connection ring 14A are not illustrated because they are perpendicular to the plane of the drawing.

The connecting portion between the clip 12A and the clip 12B is situated directly below the skirt portions 38 of the connection ring 14A, and hence, in the state as illustrated in FIG. 6B, the forward end of the clip 12B substantially coincides with the forward end of the sheath 16.

When the sheath 16 is drawn, there is exerted a frictional force between the sheath 16 and the connection rings 14A through 14E fitted into the sheath 16. However, between the connection rings 14A through 14E and the clips 12A through 12E, there are exerted the pressurizing force of the clips 12 due to the inner side portions of the closed skirt portions 38, and the pressurizing force applied to the inner wall surfaces of the connection rings 14 (second regions 34 thereof, see FIG. 3B) due to the resilient force of the claw portions 22 of the succeeding clips 12 inclined to open. Further, the projections 30 of the clips 12B through 12E abut the proximal ends of the connection rings 14A through 14D, and cannot enter the holes 43 of the connection rings 14 (see FIG. 3B). Thus, even if the sheath 16 is drawn, the connection rings 14A through 14E make no unnecessary movement. Thus, the connection rings 14A through 14E can maintain the state in which they respectively retain the clips 12A through 12E.

Next, the clipping device 10 in the state of FIG. 6B is moved to press the claw portions 22 of the diverged clip 12A against the portion to be subjected to clipping, and the lever 60 of the manipulating portion 50 (see FIGS. 5A and 5B) is pulled, whereby the manipulating wire 20 is pulled by a predetermined amount. By pulling the manipulating wire 20, the clips 12A through 12E engaged sequentially starting from the dummy clip 18 are pulled all together.

At this time, in the state of FIGS. 6B and 6C, the skirt portions 38 of the connection ring 14A protruding from the sheath 16 are open, and the pressurizing retention of the clip 12A by the skirt portions 38 is released. Further, the skirt portions 38 of the connection ring 14E are open at the forward end of the sheath 16, whereby the connection ring 14A is prevented from retreating into the sheath 16. Thus, as illustrated in FIG. 6C, the foremost clip 12A retreats relative to the connection ring 14A. The forward end of the connection ring 14A, that is, the clamping portion 40, is pushed down to a position directly below the projections 30 of the clip 12A, whereby the clamping of the clip 12A by the connection ring 14A is completed.

At the same time, the engagement portion between the clip 12A and the next clip 12B leaves the rear end of the connection ring 14A. When the engagement portion between the clip 12A and the clip 12B is detached from the connection ring 14A, the arm portions 28 are diverged by the resilient force of the clip 12B until they abut the inner wall of the sheath 16, and the claw portions 22 are opened until their interval becomes larger than the width of the turned portion 24 of the clip 12A, thereby canceling the connection between the clip 12A and the clip 12B. As a result, the clip 12A and the connection ring 14A can be detached from the sheath 16, and the clipping by the clip 12A and the connection ring 14A is completed.

On the other hand, the succeeding clips 12B through 12E are retained by the connection rings 14B through 14E whose skirt portions 38 are closed so as not to move in the rotating direction and the advancing/retreating direction with respect to the connection rings 14B through 14E. Further, the claw portions 22 are pressed against the inner walls of the second regions 34 (see FIG. 3B) of the connection rings 14B through 14E by the expanding force (urging force) of the claw portions 22 of the clips 12C through 12E engaged with the clips 12B through 12E and the claw portions of the dummy clip 18, with the result that the frictional force between the clips 12B through 12E and the connection rings 14B through 14E is enhanced. Thus, the connection rings 14B through 14E move with the movement of the clips 14B trough 14E.

That is, the clips and the connection rings other than the foremost clip 12A and the connection ring 14A retaining the same, i.e., the clips 12B through 12E and the connection rings 14B through 14E advance and retreat integrally with respect to the sheath 16, and the connected state of the clips 14B through 14E and the dummy clip 18 is maintained by the connection rings 14B through 14E.

The manipulating wire 20 is constructed so as to be capable of being pulled by a fixed amount from the initial state. This fixed amount is an amount equal to the length of the second regions 34 of the connection rings 14 or an amount slightly larger than that, and at the same time, it is an amount equal to the length from the lower ends of the projections 30 of each clip 12 to the forward end of the connection ring 14 retaining that clip 12, or an amount slightly smaller than that. In the manipulating portion 50 of FIG. 5A, this fixed amount is determined by the length as measured from the home position of the lever 60 to the movement limit at the rear.

After it has been pulled by the fixed amount, the manipulating wire 20 is soon restored by that fixed amount due to the spring 62 urging the lever 60 of the manipulating portion 50. When the pulling force of the lever 60 is canceled at the manipulating portion 50, the lever 60 is restored to the former position, and the manipulating wire 20 pulled from the state illustrated in FIG. 6B to the state illustrated in FIG. 6C is thereby restored to the former position, whereby the state as illustrated in FIG. 6D is attained. That is, as in the case of FIG. 6B, the forward end of the second clip 12B is restored to the position where it substantially coincides with the forward end of the sheath 16.

> Next, in order to place the second clip 12B in the usable state, the sheath 16 is pulled by the predetermined one stroke, that is, by the length L. In the manipulating portion 50 of FIG. 5A, the sheath manipulating handle 54 is moved by the length L from the second notch 66 to the third notch 66. As a result, the forward end of the sheath 16 is lowered to the position where the skirt portions 38 of the next connection ring 14B are opened, and the claw portions 22 of the clip 12B protruding from the sheath 16 are diverged, whereby the state as illustrated in FIG. 6E is attained.

The length L, which corresponds to one stroke by which the sheath 16 is pulled, is substantially equal to the distance between the forward ends of the two front and rear clips 12 loaded into the sheath 16, that is, a loading interval of the clips 12 in the sheath 16. The length L, which corresponds to one stroke by which the sheath 16 is pulled, is determined by the length between the notches 66 of the manipulating portion 50.

After that, as in the case of the clip 12A described above, the claw portions of the clip 12B are pressed against the portion to be subjected to clipping, and the manipulating wire 20 is pulled by a predetermined amount. As a result, the clamping of the clip 12B by the connection ring 14B is completed, and, at the same time, the connection between the clip 12B and clip 12C is canceled, whereby the clipping by the clip 12b is completed.

As described above, in the clipping device 10 of Embodiment 1, due to the connection ring 14 formed integrally by the metal clamping portion 40 and the resin retaining portion 42, it is possible, with a single component, to prevent the sheath 16 from retreating, and to secure the strong clamping force of the clip 12, that is, the requisite frictional force for clamping, while securing the requisite elasticity and rigidity for the skirt portions 38 retaining the clip 12.

Further, the clip 12 is maintained in the connected state by the connection ring 14, and hence the connection state of the clip 12 is reliably maintained. In addition, the connection portion of the clip 12 is covered with the connection ring 14, and hence there is no fear of the inner wall of the sheath 16 being flawed by a corner portion of the connecting portion of the clip 12 at the time of clipping manipulation or the like, and, when inserting the sheath 16 into the endoscope, there is very little possibility of twisting or torsion being generated in the clip 12 at the connection portion.

Further, in the clipping device 10 of Embodiment 1, the retaining portion 42 of the connection ring 14 is formed of resin, and hence the friction between the connection ring 14 and the inner wall of the sheath 16 is small, and it is possible to smoothly perform the manipulation of causing the clip 12 to advance and retreat by the manipulating wire 20 and the manipulation of pulling the sheath 16, with there being no fear of the inner wall of the sheath 16 being flawed. It is desirable for the outer diameter of the clamping portion 40 to be equal to or slightly smaller than the outer diameter of the retaining portion 42.

The sheath 16 loaded with the clips 12 has to pass a curved portion in the endoscope when being inserted into the endoscope inserted into the living body. In this regard, the retaining portion 42 is formed of resin, and hence it is superior in flexibility, and can be bent while retaining the connecting portion of the clips 12.

In the state in which the clips are set in the sheath 16, the skirt portions 38 of the retaining portions 42 of the connection rings 14 retain the clips 12 through pressurization, and hence it is possible to retain the connecting portions of the clips 12 in a fixed state, and there is very little play in the connecting portions. Thus, the advancing/retreating movement at the time of manipulation by the manipulating wire 20 is stabilized, and the error in the movement amount is small, making it possible to effect movement with high precision.

By pulling the dummy clip 18 and the plurality of clips 12 connected thereto by a predetermined length in one direction by the manipulating wire 20, it is possible to effect the clamping of the foremost clip 12 by the clamping portion 40 of the connection ring 14 and the canceling of the connection with the next clip 12, and hence it is possible to perform the clipping by the foremost clip 12. Further, by pulling the sheath 16 toward the manipulating portion side by the predetermined length L, the next clip 12 becomes usable, thus making it possible to continue clipping.

While in the above-mentioined example the clips 12 are connected together with their orientations alternately changed by 90 degrees, this should not be construed restrictively, and the inner configuration of the connection clip can be selected according to the configuration of the engagement portion. For example, it is also possible to adopt a clip of a configuration in which twisting is effected by 90 degrees at the portion between the claw portions 22 and the turned portion 24, connecting together the consecutive clips in the same orientation. Further, by using a closed clip with a turned portion, it is advantageously possible to impart a resilient force pressurizing the turned portion and diverging the arm portions. The present invention, however, is also applicable to a construction adopting an open clip (U-shaped clip) with no turned portion.

Next, a package for the clips 12 used in the clipping device 10 is described.

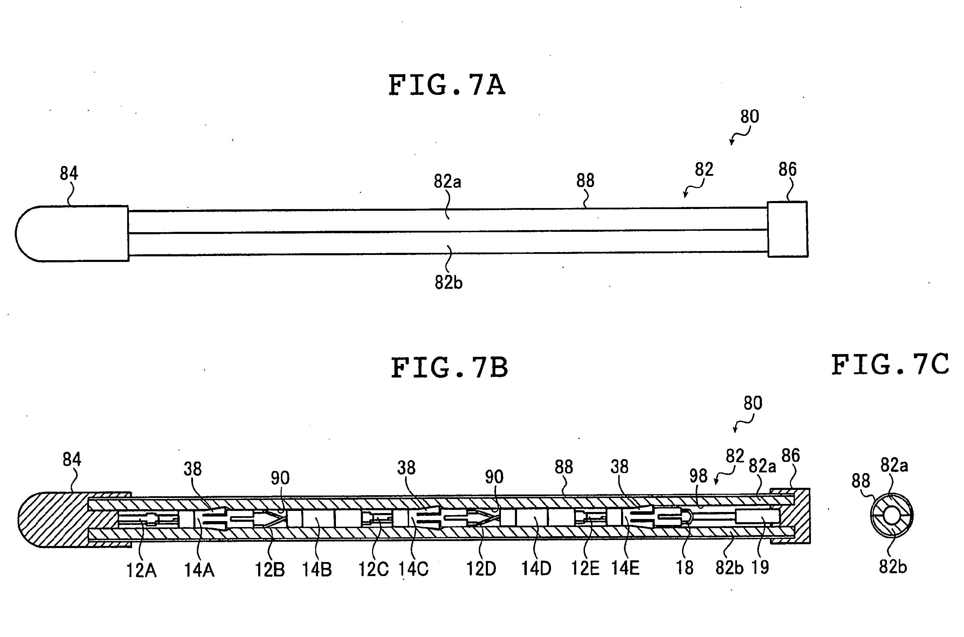

FIGS. 7A through 7C illustrate a connection clip package 80. In the connection clip package 80, a predetermined number of clip units (clips 12 with the connection rings 14 fitted thereto) to be used in the above-mentioned magazine type clipping device 10 are previously connected together and accommodated in the same manner as when they are loaded into the sheath 16, thus forming a package. FIG. 7A is a front view, FIG. 7B is a sectional view, and FIG. 7C is a sectional view taken along a plane orthogonal to the axis of the case. In the following, the left-hand side in FIGS. 7A and 7B is referred to as the forward end, and the right-hand side thereof as the rear end.

As illustrated in FIG. 7A, the connection clip package 80 includes a case 82, a top cap 84, and a bottom cap 86.

The case 82 is of a cylindrical configuration, and accommodates therein the clip units including the clips 12 and the connection rings 14. As illustrated in FIGS. 7A and 7C, the case 82 is formed by combining two case components 82a and 82b which are semi-cylindrical and substantially of the same configuration. The top cap 84 is fitted onto the forward ends of the two case components 82a and 82b, and the bottom cap 86 is fitted onto the rear ends thereof, maintaining the case 82 in the closed state.

It is desirable for the case 82 to be transparent or translucent so that its interior can be seen. Further, from the viewpoint of impact resistance, ease of handling, and ease of molding, it is desirable for the case to be formed of a resin that is not deteriorated in the fluctuation range of the ambient temperature (e.g., 5°C to 38°C). While in this embodiment the case 82 is formed in a cylindrical configuration, the outer configuration of the case 82 is not restricted to a columnar one, and it may also be of a prism-like configuration.

The connection clip package 80 accommodates medical clips, and hence it is necessary to maintain the interior of the case 82 in a hermetically sealed condition. For this purpose, in the case 82, the outer surfaces of the case components 82a and 82b are covered with a cover 88 made of a transparent resin, thus securing the airtightness of the interior of the case 82. Alternatively, it is also possible to form the case components 82a and 82b of the case 82 of an elastic material, and to keep the mating surfaces of the case components 82a and 82b pressed against each other by the top cap 84 and the bottom cap 86, thereby securing the airtightness. It is also possible to provide packing between the case components 82a and 82b to secure the requisite airtightness.

There are no particular limitations regarding the top cap 84 and the bottom cap 86 as long as they can hermetically seal the case components 82a and 82b, and they may be formed of rubber or resin. The bottom cap 86 is detachable. When loading the clip units in the case 82 into the sheath, the bottom cap 86 is removed, and the clip units therein are drawn out while in the connected state. It does not matter whether the top cap 84 is detachable or not. Further, it is also possible to form the forward end portion by the case 82, without providing any top cap 84.

As illustrated in FIG. 7B, there is formed in the case 82 a hole whose inner diameter is slightly larger than the outer diameter of the connection rings 14 and substantially equal to the inner diameter of the sheath into which the clip units are loaded, with the hole extending through the entire case 82. The five clips 12A through 12E connected together and the dummy clip 18 and the five connection rings 14A through 14E covering the connecting portions thereof are accommodated in the hole. The forward end of the foremost clip 12A is protected by the portion protruding from the top cap 84 into the case 82. The connecting member 19 at the rear end of the dummy clip 18 connected to the rearmost clip 12E is retained by the bottom cap 86.

At the rear end portion of the case 82, there is formed a sheath fit-engagement portion 98 into which the sheath can be inserted. The sheath fit-engagement portion 98 has a diameter substantially equal to the outer diameter of the sheath into which the clips 12A through 12E and the connection rings 14A through 14E are loaded. The diameter of the sheath fit-engagement portion 98 is larger than the diameter of a straight portion 90 of the hole of the case 82 by approximately the thickness of the sheath, and hence there is a corresponding step at the forward end of the sheath fit-engagement portion 98. When loading the clip units in the case 82 into the sheath, the sheath is inserted up to the forward end of the sheath fit-engagement portion 98.

The forward end of the sheath fit-engagement portion 98 is substantially at the same position as the forward end of the dummy clip 18 accommodated in the case 82, that is, substantially at the same position as the rear end of the rearmost clip 12E, and it is situated directly below the skirt portions 38 of the connection ring 14E.

FIG. 8 is a partial enlarged view of FIG. 7B. As illustrated in FIG. 8, in the inner surface of the case 82, there are formed, at the positions where the connection rings 14A through 14E are accommodated, recesses 96 corresponding to the configuration of the skirt portions 38. Each recess 96 is formed by a first inclined portion 92 expanding radially outwards from the straight portion 90 at substantially the same angle of the inclination of the skirt portions 38 in the natural state so as to be substantially in conformity with the expansion of the skirt portions 38, and a second inclined portion 94 radially narrowed from the expanded end portion (rear end) of the first inclined portion 92.

As described above, the clips 12A through 12E are connected together, with their orientations alternately changed by 90 degrees, and, in correspondence therewith, the connection rings 14A through 14E are fitted onto the clips 12A through 12E with their orientations alternately changed by 90 degrees. Thus, the positions of the recesses 96 of the case 82 are also deviated from each other by 90 degrees in the circumferential direction at positions corresponding to the connection rings 14A through 14E. As illustrated in FIG. 7B, two upper and lower recesses 96 are provided for each recess 96 corresponding to the skirt portions 38 of each of the connection rings 14A, 14C, and 14E. Two recesses are formed in a direction perpendicular to the plane of FIG. 7B for each recess 96 corresponding to the skirt portions 38 of each of the connection rings 14B and 14D.

It is also possible for the recesses 96 to be formed over the entire periphery at the positions corresponding to the skirt portions 38 in the longitudinal direction (the horizontal direction as seen in the drawing).

Due to the first inclined portions 92 of the recesses 96, the connection rings. 14A through 14E are accommodated in the case 82 with their skirt portions 38 being in the diverged state without receiving any external force. Thus, it is possible to prevent the elasticity of the skirt portions 38 from deteriorating while stored in the case 82, thus making it possible to maintain the performance of the connection rings 14A. through 14E.

When the clips 12A through 12E and the connection rings 14A through 14E are drawn out of the case 82, the skirt portions 38 open in the recesses 96 are gradually closed while guided by the second inclined portions 94, and hence they are not turned up when they leave the recesses 96, and can move within the case 82 while accommodated in the straight portion 90.

The clip units are accommodated in the case 82 as follows.

First, the clips 12A through 12E are successively connected together. In connecting the clips 12A through 12E, the turned portion 24 of one clip 12 is engaged with the claw portions 22 of the next clip 12, and the engagement portion is set at a predetermined position on the connection ring 14. The last clip 12E is connected to the dummy clip 18 in a similar manner.

The connection clips previously connected together and assembled in the state in which they are to be loaded into the sheath 16 are accommodated in one case component 82a of the case 82. After that, the other case component 82b is put on the case component 82a, and the top cap 84 and the bottom cap 86 are fitted, whereby the connection clip package 80 is obtained.

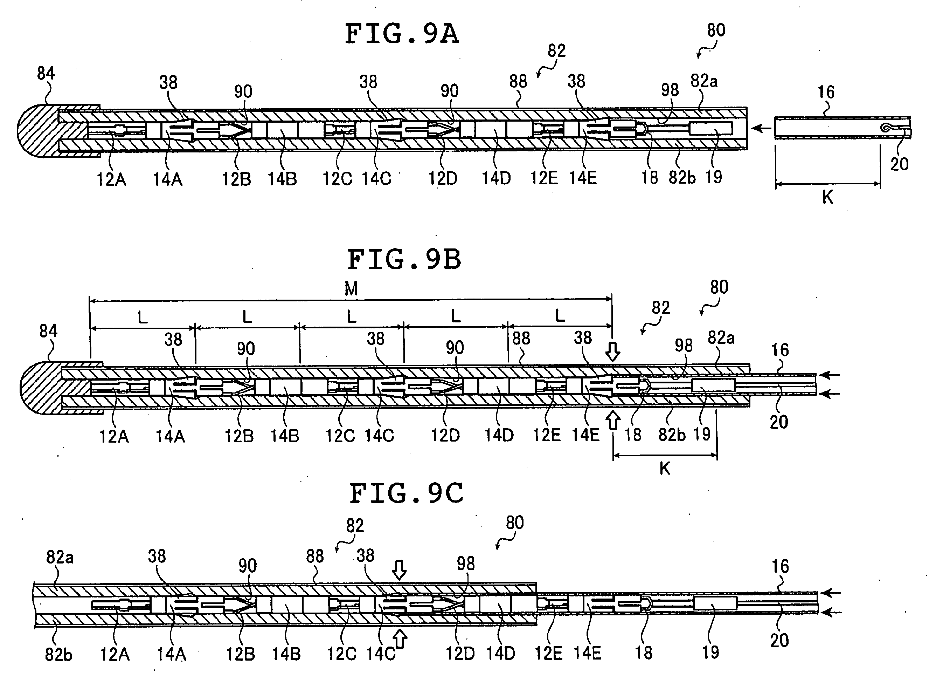

Next, a method of loading the clip units into the sheath 16 from the connection clip package 80 is described with reference to FIGS. 9A through 9C.

Prior to the loading of new clip units, the dummy clip 18 that has been engaged with the rearmost clip 12E already used is removed from the manipulating wire 20.

When, in the clipping device 10 described above, all the clips 12 have been used, the forward end of the dummy clip 18 substantially coincides with the forward end of the sheath 16. In the manipulating portion 50, the claw 76 of the sheath manipulating handle 54 is on the sixth notch 66 after the five clips have been used. When, in this state, the sheath manipulating handle 54 is further pulled toward the wire manipulating handle 52 side, the sheath 16 is caused to retreat, and the dummy clip 18 protrudes from the forward end of the sheath 16, making it possible to detach the dummy clip 18 from the manipulating wire 20.

When, with the dummy clip 18 being removed, the sheath manipulating handle 54 is restored to the former position, that is, the position where the claw is on the sixth notch 66, the forward end of the manipulating wire 20 is retracted from the forward end of the sheath 16 by a length K as illustrated in FIG. 9A.

On the other hand, in the case 82, the length in the depth direction of the sheath fit-engagement portion 98 is determined such that the length as measured from the position where the forward end of the manipulating wire 20 connected to the connecting member 19 is situated to the forward end of the sheath fit-engagement portion 98 is K. In this way, the positional relationship between the sheath 16 before fit-engagement with the case 82 and the manipulating wire 20 is maintained also after the sheath 16 and the case 82 have been fit-engaged with each other, and hence, when the sheath 16 is fit-engaged, it is possible to prevent any surplus force such as the tensile force due to manipulating wire 20 from being applied to the clips 12A through 12E in the case 82.

First, as illustrated in FIG. 9A, when loading the clip units, the bottom cap 86 of the connection clip package 80 is removed, and the manipulating wire 20 protruding from the forward end of the sheath 16 is connected to the connecting member 19 at the rear end of the dummy clip 18 in the case 82.

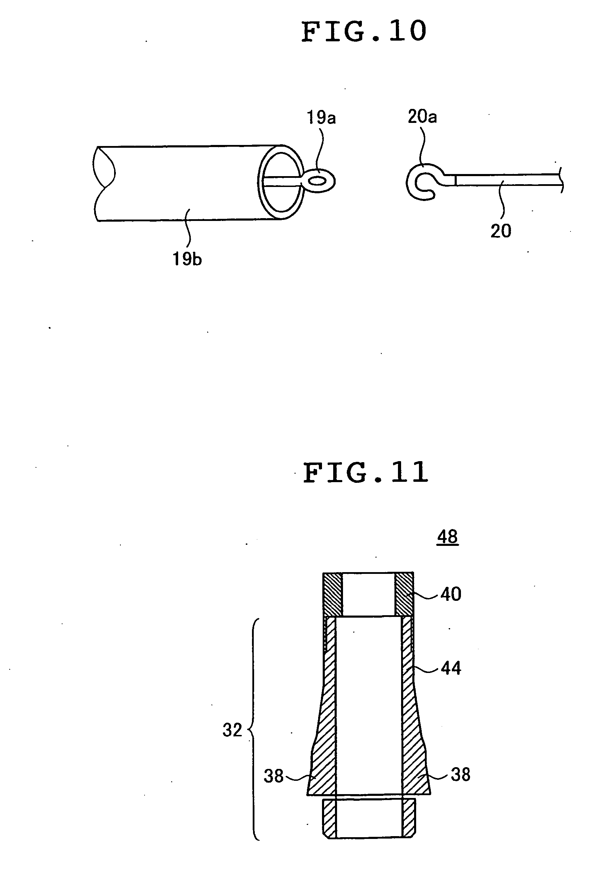

As illustrated in FIG. 10, the connecting member 19 at the rear end of the dummy clip 18 has a connection ring 19a and a cover 19b. At the time of connection with the manipulating wire 20, the connection ring 19a is drawn out of the cover 19b, and is connected to the manipulating wire 20, and then the connecting portion thereof is covered with the cover 19b.

A hook-like member 20a is attached to the forward end of the manipulating wire 20. The hook-like member 20a of the manipulating wire 20 is hooked onto the connection ring 19a of the connecting member 19 to connect the dummy clip 18 and the manipulating wire 20 to each other.

The connecting portion of the hook-like member 20a and the connection ring 19a is protected against detachment by being covered with the cover 19b.

When the manipulating wire 20 is connected to the dummy clip 18 in the case 82, the operator holds the sheath 16 and the case 82, and inserts the end portion of the sheath 16 into the sheath fit-engagement portion 98 of the case 82. Then, the sheath manipulating handle 54 of the manipulating portion 50 (see FIGS. 5A and 5B) is caused to advance with respect to the wire manipulating handle 52, whereby the sheath 16 is caused to advance with respect to the manipulating wire 20, and the sheath 16 is inserted up to the forward end of the sheath fit-engagement portion 98.

In order to connect the manipulating wire 20 to the dummy clip 18, the sheath manipulating handle 54 that has been drawn to the wire manipulating handle 52 side, is restored to the former position, that is, the position where it is engaged with the sixth notch 66, whereby, as illustrated in FIG. 9B, it is possible to insert the sheath up to the forward end of the sheath fit-engagement portion 98 to fit-engage it with the case 82.

It is also possible to adopt some other construction as long as the connecting member 19 (rear end portion of the dummy clip 18) and the forward end of the manipulating wire 20 are detachable with respect to each other and no detachment occurs as a result of the advancement/retreating movement of the manipulating wire 20.

In order to enhance the fit-engagement force exerted between the sheath 16 and the case 82 to prevent detachment of the sheath 16 and the case 82 from each other during clip loading manipulation, a minute protrusion may be imparted to one or both of the outer surface of the sheath 16 and the surface of the sheath fit-engagement portion 98. Due to the provision of such a minute protrusion, the fit-engagement portion can be placed in a lightly press-fitted state. Further, it is possible to enhance the frictional force to reliably maintain the fit-engagement state.

In the state in which the sheath 16 has been fit-engaged with the sheath fit-engagement portion 98 of the case 82, the inner diameter of the straight portion 90 of the case 82 and the inner diameter of the sheath 16 are substantially equal to each other.

Next, as illustrated in FIGS. 9B and 9C, solely the sheath 16 is moved toward the forward end side by a length M, with the manipulating wire 20 remaining at the same position, and, with that, the case 82 is moved toward the forward end side by the length M. Through the movement of the sheath 16 and the case 82, the clips 12A through 12E and the connection rings 14A through 14E in the case 82 are successively loaded into the sheath 16 starting with the rear end side.

The movement of the sheath 16 is effected by moving the sheath manipulating handle 54 of the manipulating portion 50 to the forward end side with respect to the wire manipulating handle 52. In the manipulating portion 50 of FIGS. 5A and 5B, the sheath manipulating handle 54 is caused to slide forwards at one time by the length M with respect to the wire manipulating handle 52 from the state in which the hook 76 is engaged with the sixth notch 66 as from the front side to the position where it is engaged with the first notch 66. The length M is equal to the sum total of the five intervals L between the notches 66.

That is, the movement length M of the sheath 16 at the time of clip loading is equal to a length obtained by multiplying the loading interval L of the clips 12 of the sheath 16, that is, the amount L by which the sheath 16 is caused to retreat each time one clip 12 is used, by the number of clips 12 connected together for use in the clipping device 10.

As described above, in the manipulating portion 50, the use of the connection clips is started from the state in which the claw 76 is hooked onto the first notch 66 as from the front side, and, each time one clip 12 is used, the sheath manipulating handle 54 is caused to slide to the next notch 66 on the rear side, whereby the sheath 16 is caused to retreat, and the clipping device is placed in the state in which the next clip 12 can be used. This operation is repeated the number of times corresponding to the number of clips 12 loaded (which is five in this example), and, in the state in which all the clips 12 have been used, the sheath manipulating handle 54 moves to the sixth notch 66 to approach the wire manipulating handle 52 side. Thus, the forward end of the manipulating wire 20 has advanced toward the forward end of the sheath 16.

At the time of loading of new connection clips, the manipulating wire 20 and the clip row of the connection clip package 80 are connected together with all the clips 12 having been used, the sheath 16 is restored forward by the amount it has moved at the time of use, that is, L multiplied by 5 = M, whereby it is possible to load a new clip row in the same condition as the former clip row. That is, the forward end of the foremost clip 12 of the newly loaded clip row is placed at a position substantially coinciding with the forward end of the sheath 16.

When moving the sheath 16 toward the forward end of the connection clip package 80 through manipulation of the manipulating portion 50, it is desirable to move the sheath 16 and the case 82 while pressing the portion in the vicinity of the forward end of the sheath fit-engagement portion 98 indicated by the arrow in FIGS. 9B and 9C. When the sheath 16 and the case 82 move with respect to the clips 12 connected to the manipulating wire 20 and the connection rings 14, a pulling force is exerted on the clip row formed of the clips 12A through 12E and the dummy clip 18 connected together. However, by moving the sheath 16 and the case 82 while pressing the portion in the vicinity of the forward end of the sheath fit-engagement portion 98, it is possible to suppress the pulling force applied to the clips 12A through 12E to a low level, thus making it possible to prevent troubles such as deviation of the interval of the clips 12, detachment of the connecting portion from the connection rings 14, and deformation of the clips 12.

Further, as illustrated in FIG. 9B, the forward end of the sheath fit-engagement portion 98 is directly below the recess 96 (see FIG. 8) accommodating the skirt portions 38 of the connection ring 14E, and hence, by pressing this portion in the vicinity of the forward end, it is possible to smoothly close the skirt portions 38 staying in this recess 96 or passing this recess 96.

In the case 82, the skirt portions 38 of the connection rings 14A through 14E are accommodated in the recesses 96 in the open state. However, when the case 82 moves toward the forward end, the skirt portions 38 are closed while guided by the second inclined portions 94 of the recesses 96, and accommodated in the straight portion 90 to be drawn into the sheath 16 as they are. The skirt portions 38 of the connection rings 14A through 14C pass the other recesses 96 on the rear end side. However, if, in this process, the skirt portions 38 thereof are once opened at the first inclined portions 92, they are closed again at the second inclined portions 94, and are guided to the straight portion 90.

The inner walls of the second regions 34 (see FIG. 3B) on the rear side of the connection rings 14A through 14E are pressurized by the urging force with which the claw portions 22 of the succeeding clips 12B through 12E and the dummy clip 18 are inclined to be diverged. Thus, in the case 82, the connected state of the clips, and the positional relationship between the clips 12A through 12E and the connection rings 14A through 14E are maintained.

Further, when loading the clips into the sheath 16, the skirt portions 38 move from the recesses 96 to the straight portion 90 to be thereby closed, whereby the clips 12A through 12E in the connection rings 14A through 14E are pressurized by the inner side portions of the skirt portions 38, and the connection rings 14A through 14E maintain the clips 12A through 12E and the dummy clip 18 in the connected state. Thus, at the time of loading of the clips into the sheath 16, it is possible to prevent disengagement of the clips 12A through 12E and the dummy clip 18 and deviation in their positional relationship with the connection rings 14A through 14E.

As a result of the movement of the sheath 16 by the length M, the forward end of the sheath 16 moves to the position where it accommodates the forward end of the foremost clip 12A, whereby the loading of the clips into the sheath 16 is completed. At the time of completion of the loading, in the manipulating portion 50 (see FIGS. 5A and 5B), the sheath manipulating handle 54 moves toward the forward end, and the claw 76 is hooked onto the first notch 66.

In this way, the connection clip package 80 allows distribution and storage in the state in which the clips are connected together. Further, it allows loading of the clips into the sheath 16 by a simple manipulation while maintaining the connected state. Thus, the operational burden on the operator is small, and the loading of the clips can be effected easily in a short time.

Further, solely by performing the above-mentioned loading manipulation, the newly loaded clips can be placed at predetermined positions in the sheath 16. Thus, there is no need to perform fine adjustment on, for example, the amount by which the clip 12 protrudes from the sheath 16 during clipping manipulation, thus facilitating the clipping manipulation.

While in Embodiment 1 described above the dummy clip 18 and the manipulating wire 20 are detachably connected via the connecting portion 19, this should not be construed restrictively, and it is also possible to fixedly connect the manipulating wire 20 to the dummy clip 18.

### Embodiment 2

Next, Embodiment 2 of the present invention is described. While the clipping device of Embodiment 1 described above is of the magazine type, the clipping device of Embodiment 2 is of a single-loader type. FIG. 11 illustrates a retaining ring 48 used in the clipping device of Embodiment 2.

The retaining ring 48 includes a clamping portion 40 similar to the clamping portion 40 of the connection ring 14 used in Embodiment 1, and a retaining portion 44 formed by shortening the retaining portion 42 and substantially formed solely of the first region 32. The construction and operation of the clamping portion 40 are the same as those of the clamping portion 40 of the connection ring 14 of Embodiment 1, and the construction and operation of the retaining portion 44 are the same as those of the first region 32 of the retaining portion 42 of the connection ring 14 of Embodiment 1.

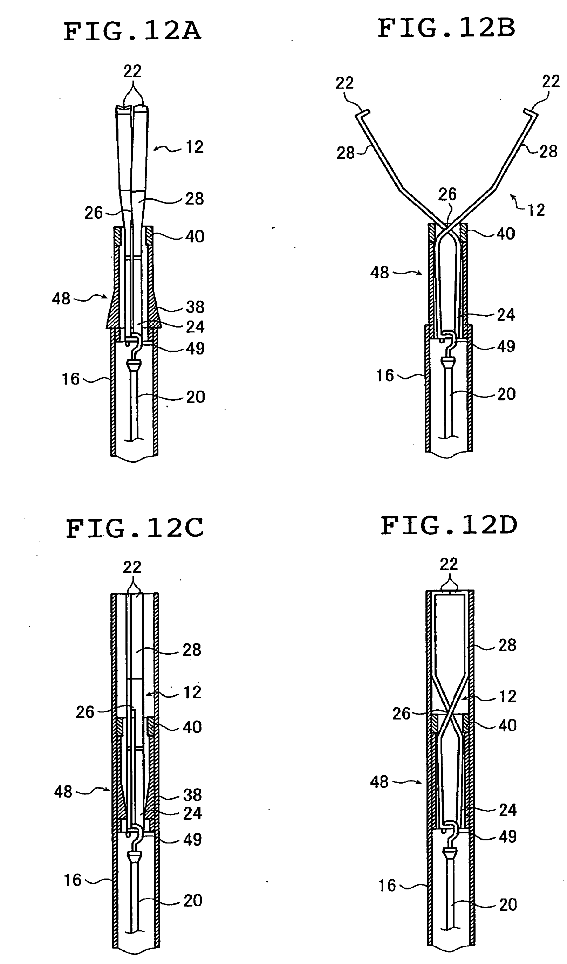

FIGS. 12A through 12D illustrate a clipping device 46 according to Embodiment 2. FIGS. 12B and 12D are views as seen from an angle differing by 90 degrees from FIGS. 12A and 12C, respectively.

The clipping device has one clip 12, a retaining ring 48 retaining the clip 12, a hook 49 engaged with the clip 12, and the manipulating wire 20 connected to the hook 49. Those components are fitted into the sheath 16.

As illustrated in FIGS. 12C and 12D, a bleeding stop clip unit including the clip 12 and the retaining ring 48 is loaded into the forward end portion of the sheath 16. The loading of the bleeding stop clip unit is conducted, for example, as follows: the retaining ring 48 is previously fitted onto the clip 12, and the hook 49 is engaged with the turned portion 24 of the clip 12; the hook 49 is attached to the forward end of the manipulating wire 20 protruding from the forward end of the sheath 16, and then the sheath 16 is caused to advance relative to the manipulating wire 20 to accommodate the clip 12 in the sheath 16.

When solely the sheath 16 is pulled by a predetermined amount toward the manipulating portion with the manipulating wire 20 remaining as it is, the forward end of the sheath 16 is lowered to a position where the skirt portions 38 of the retaining ring 48 are opened, and the claw portions 22 of the clip 12 are diverged to attain the state as illustrated in FIGS. 12A and 12B. When, in this state, the manipulating wire 20 is pulled, the clip 12 retreats with respect to the sheath 16 and the retaining ring 48 that has become incapable of retreating due to the skirt portions 38 opened, and the clamping portion 40 of the retaining ring 48 is forced into the forward end portion of the clip 12, whereby the clamping of the clip 12 by the retaining ring 48 is completed. At the same time, the engagement portion of the clip 12 and the hook 49 leaves the rear end of the retaining ring 48, and the engagement of the clip 12 and the hook 49 is canceled, whereby the clipping by the clip 12 is completed.

The hook 49 may be formed so as to undergo plastic deformation upon a fixed level of pulling force. After clamping the clip 12 by the retaining ring 48, the manipulating wire 20 is further pulled to apply a force larger than the above-mentioned fixed level of pulling force, whereby the hook 49 is deformed, and its engagement with the clip 12 is canceled.

After performing clipping one time, the sheath 16 is drawn out of the endoscope, and the next bleeding stop unit is attached to the forward end of the manipulating wire, whereby it is possible to perform next clipping.

### Embodiment 3

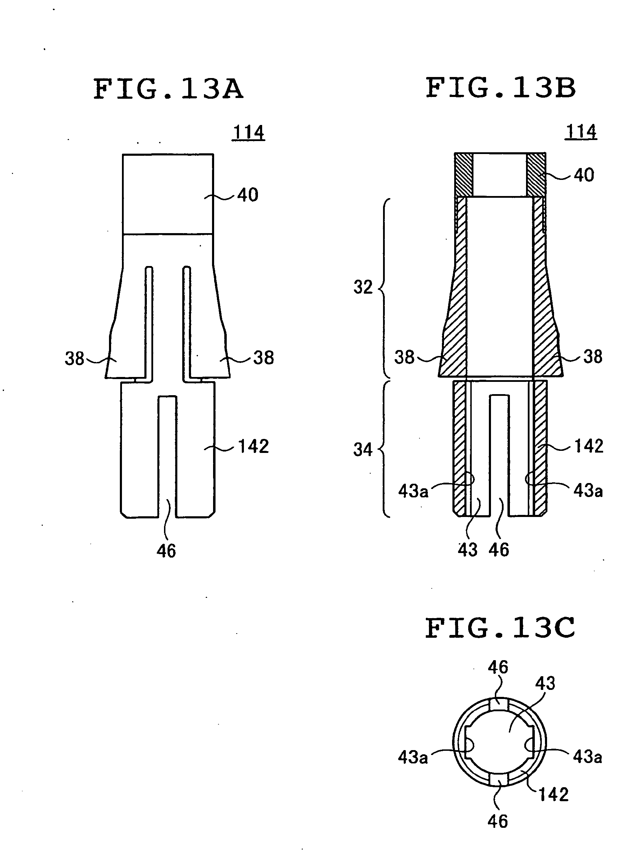

Instead of the connection rings 14 of the clipping devices 10 of Embodiment 1, it is possible to use a connection ring 114 as illustrated in FIGS. 13A through 13C.

The connection ring 114 is of the same construction as the connection ring 14 of Embodiment 1 except that two slits 46 are formed in a second region 134 of a retaining portion 142. That is, the connection ring 114 includes the metal clamping portion 40 and the resin retaining portion 142, and the retaining portion 142 has a first region 32 and a second region 134, with the second region 134 having the slits 46 cut from the proximal end thereof at positions opposed to each other.

The slits 46 are formed at two positions deviated from the skirt portions 38 by 90 degrees so as to be shallower than the upper end of the second region 134. In other words, the slits 46 are provided at positions deviated by 90 degrees from the direction in which the clips 12 retained by the second region 134 are diverged.

Due to the provision of the slits 46, the connection ring 114 is improved in terms of flexibility, and the clipping device 100 can pass a curved portion of small curvature. Further, due to the provision of the slits 46, the hem (proximal end portion) of the connection ring 114 is partially turned up, and hence, when the front and rear clips 12 are connected together prior to the loading of the clips 12 into the sheath 16, the connection is advantageously facilitated through the turning of the hem of the connection ring 114.

The slits 46 are situated so as to be shallower than the skirt portions 38, whereby a substantial reduction in the strength of the connection ring 114 is prevented. Further, the depth of the slits 46 is shallower than the position of the rear end of the clip 12 retained in the first region 32, that is, shallower than the engagement position of the clips 12, and hence, also in the connection clip unit prior to the loading into the sheath 16, it is possible to maintain the retention of the clip 12 in the second region 134 of the connection ring 114.

### Embodiment 4

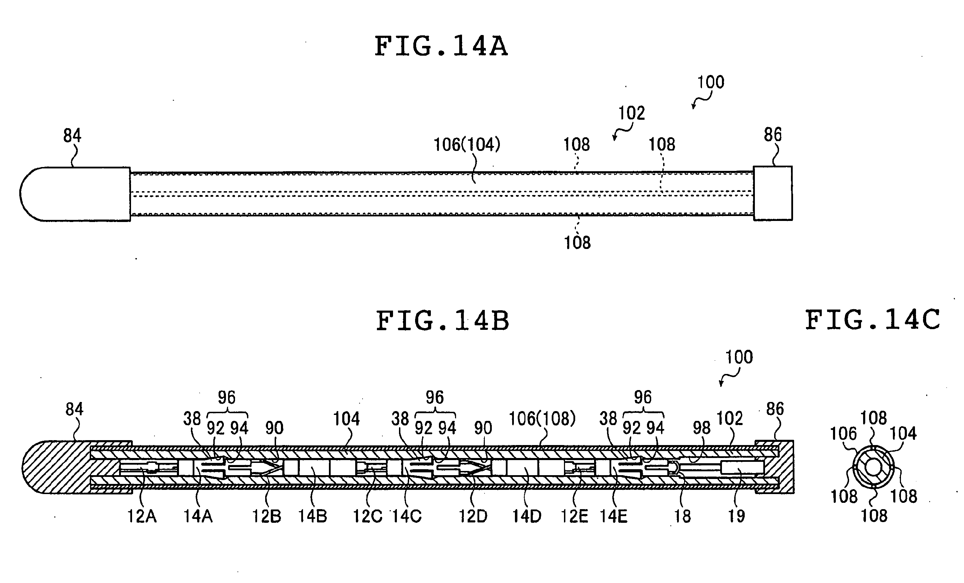

A connection clip package 100 according to Embodiment 4 is described with reference to FIGS. 14A through 14C. The connection clip package 100 has a case 102 that is of a double structure formed of an inner cylinder 104 and an outer cylinder 106. A top cap 84 fitted onto the forward end of the inner cylinder 104 and the outer cylinder 106 and a bottom cap 86 fitted onto the rear end thereof are similar the top cap 84 and the bottom cap 86 of the connection clip package 80 described above.

The inner cylinder 104 is a cylindrical member formed of an elastic material. The configuration of the hole of the inner cylinder 104 is the same as the configuration of the hole of the case 82 of the connection clip package 80 described above. The straight portion 90 has recesses 96 formed at a position corresponding to the skirt portions 38 of the connection rings 14A through 14E. Formed at the rear end portion of the inner cylinder 104 is the sheath fit-engagement portion 98 whose diameter is slightly larger than that of the straight portion 90 and substantially equal to the outer diameter of the sheath 16.

The outer cylinder 106 is a cylindrical member covering the inner cylinder 104. The outer cylinder 106 exhibits elasticity and has a plurality of bar members 108 arranged parallel to the axial direction thereof. As illustrated in FIG. 14C, four bar members 108 are arranged at an interval of 90 degrees. The bar members 108 are arranged in correspondence with the positions of the skirt portions 38 of the connection rings 14A through 14E, that is, the positions of the recesses 96 of the inner cylinder 104.

The top cap 84 and the bottom cap 86 are fitted to both ends of the bar members 108, whereby the radial position of the case 102 is fixed. The bar members 108 support the case 102 during distribution and storage so that the case 102 (inner cylinder 104 and outer cylinder 106) formed of an elastic material may not be bent or crushed under an external force, thus protecting the clip units therein against deformation and breakage.

When the bottom cap 86 are pressurized with the bottom cap 86 being removed, the bar members 108 presses the case 102 as a whole in the axial direction to reduce in diameter thereof.

When loading the clips of the connection clip package 100 into the sheath 16, the bottom cap 86 is removed as in the case of the connection clip package 80 of Embodiment 1, and the manipulating wire 20 is connected to the connecting member 19 at the rear end of the dummy clip 18. Then, the forward end of the sheath 16 is inserted into the interior of the case 102 to be fit-engaged with the sheath fit-engagement portion 98, and the manipulating wire 50 is manipulated to move the sheath 16 and the case 102 toward the forward end with respect to the manipulating wire 20 while pressing the sheath fit-engagement portion 98 by the operator's hand.

Here, by pressing the four bar members 108, the case 102 as a whole is pressurized in the axial direction from four directions. As a result, the case 102 formed of an elastic material is crushed, and the interval between the opposing recesses 96 is reduced, whereby the interval between the opposing recesses 96 is reduced, and the skirt portion 38 accommodated in the recesses 96 are closed. When, in this state, the sheath 16 and the case 102 are caused to advance, the skirt portions 38 can be more smoothly closed, making it possible to load the clips 12A through 12E and the connection rings 14A through 14E smoothly into the sheath 16.

It is also possible to press the bar members 108 with the top cap 84 also being removed. In this case, the case 102 is pressurized substantially uniformly in the axial direction. Further, of the four bar members 108, it is also possible for the two opposing ones to be pressed alternately.

In another method, when high elasticity is imparted to the inner cylinder 104 and the outer cylinder 106, and the case 102 is deformed to a sufficient degree by pressing the bar members 108, thereby closing the skirt portions 38 of the connection rings 14A through 14E, and when the sheath 16 can be inserted into the inner cylinder 104, the sheath 16 may be caused to enter the gap between the inner cylinder 104 and the clips 12A through 12E and the connection rings 14A through 14E, with the case 102 being pressed by the bar members 108, thereby loading the clips into the sheath 16.

In this case, there is no need to provide the second inclined portions 94 to the recesses 96. For example, the rear ends of the first inclined portions 92 may be formed as surfaces substantially perpendicular to the axial direction like the skirt portions 38.

Also in the connection clip package 100, distribution and storage are possible with the clips being connected together. Further, it is possible to load the clips into the sheath 16 while maintaining the connected state.

### Embodiment 5

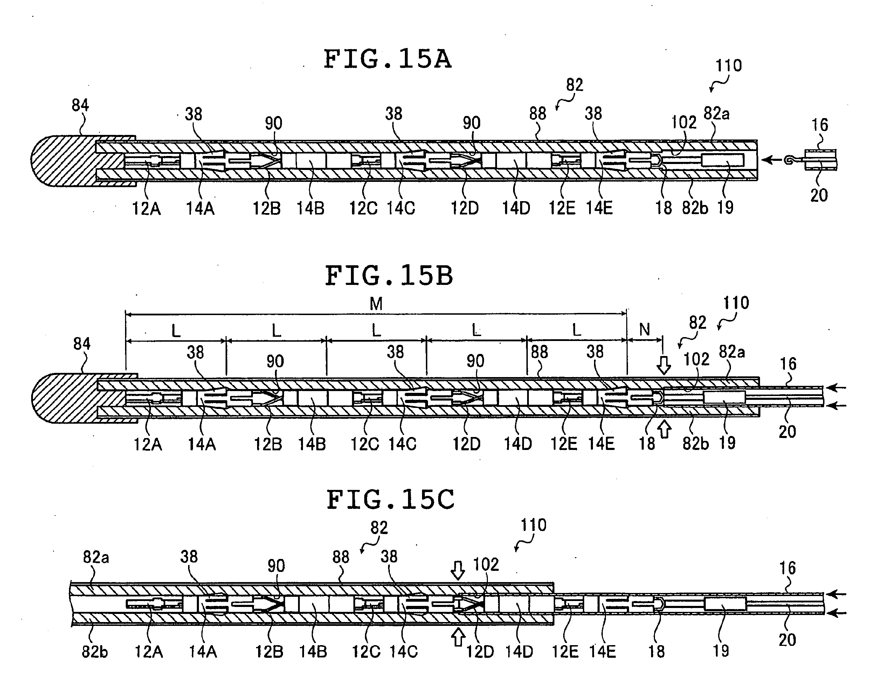

Next, a connection clip package 110 according to Embodiment 5 is described with reference to FIGS. 15A through 15C. The connection clip package 110 is of the same construction as the connection clip package 80 except that its sheath fit-engagement portion 102 is formed up to a position shallower than that of the sheath fit-engagement portion 98 of the connection clip package 80 of Embodiment 1. In the connection clip package 110, the same components as those of the connection clip package 80 are indicated by the same reference numerals, and a detailed description thereof is omitted.

In the case 82 of the connection clip package 110, the sheath fit-engagement portion 102 is provided to extend to a position on the rear side of the forward end of the accommodated dummy clip 18 and in the vicinity of the terminal end of the resilient member.

A method of loading clip units into the sheath 16 from the connection clip package 110 is described with reference to FIGS. 15A through 15C.

First, as illustrated in FIG. 15A, the bottom cap 86 of the connection clip package 110 is removed, and the manipulating wire 20 protruding from the forward end of the sheath 16 is connected to the connecting member 19 at the rear end of the dummy clip 18 in the case 82.

The manipulating wire 20 can be caused to protrude from the sheath 16 through manipulation of the manipulating portion 50 illustrated in FIGS. 5A and 5B. That is, in the clipping device of FIGS. 1A and 1B, after all the clips 12 have been used, the sheath manipulating handle 54 of the manipulating portion 50 illustrated in FIGS. 5A and 5B is moved to the wire manipulating handle 52 side. For example, the claw 76 of the sheath manipulating handle 54 is engaged with the rearmost notch 66. In this state, there is a predetermined interval between the wire manipulating handle 52 and the sheath manipulating handle 54, and, by pulling the sheath manipulating handle 54 by this interval, the sheath 16 is drawn with respect to the manipulating wire 20, whereby it is possible to cause the manipulating wire 20 to protrude from the forward end of the sheath 16. It is also possible to provide the positioning pipe 56 with a notch at a position corresponding to the position where the wire manipulating wire 20 is caused to protrude.

The dummy clip 18 that has been engaged with the rearmost portion of the clip 12 already used is previously removed, with the manipulating wire 20 protruding from the sheath 16.

As illustrated in FIG. 15B, when the manipulating wire 20 has been connected to the dummy clip 18 in the case 82, the sheath 16 is inserted up to the forward end of the sheath fit-engagement portion 102 to be fit-engaged with the case 82. By causing the sheath manipulating handle 54 of the manipulating portion 50 to advance with respect to the wire manipulating handle 52, it is possible to cause the sheath 16 to advance with respect to the manipulating wire 20.

With the sheath 16 having been inserted up to the forward end of the sheath fit-engagement portion 102, the positional relationship between the sheath 16 and the manipulating wire 20 is such that the sheath 16 has retreated by a length N, with the condition in which all the clips 12 have been used being a reference. In the manipulating portion 50, the sheath manipulating handle 54 has further moved to the wire manipulating handle 52 side by the length N from the position where the claw is hooked onto the sixth notch 66.

Next, as illustrated in FIGS. 15B and 15C, in this state, solely the sheath 16 is moved to the forward end side, with the manipulating wire 20 remaining as it is, and, with that, the case 82 is moved to the forward end side. The movement of the sheath 16 is effected by moving the sheath manipulating handle 54 of the manipulating portion 50 to the forward end side with respect to the wire manipulating handle 52. In the manipulating portion 50 of FIGS. 5A and 5B, the sheath manipulating handle 54 is caused to slide at one time with respect to the wire manipulating handle 52 by a length M + N to the position where the first notch 66 is engaged with the claw 76 from the state in which the claw 76 has been retracted by the length N from the sixth notch 66 as from the front side.

At this time, as in the case of the connection clip package 80 described above, it is desirable to move the sheath 16 and the case 82 while pressing the portion in the vicinity of the sheath fit-engagement portion 102 indicated by the arrow in FIGS 15B and 15C. As a result of the movement of the sheath 16 and the case 82, the clips 12A through 12E and the connection rings 14A through 14E in the case 82 are sequentially loaded into the sheath 16 starting from the rear end side.

As a result of the movement of the sheath 16 by the length M+N, the forward end of the sheath 16 moves to the position where it accommodates the forward end of the foremost clip 12A, whereby the loading of the clips into the sheath 16 is completed. At the time of completion of the loading, in the manipulating portion 50, the sheath manipulating handle 54 moves toward the forward end, and the claw 76 is hooked onto the first notch 66.

While in the examples described above the loading is effected with positioning being effected such that the forward end of the foremost clip 12A substantially coincides with the forward end of the sheath 16, it is also possible for the foremost clip 12A to be set at a position retracted from the forward end of the sheath 16 by a predetermined amount. In this case, the distance between the first notch 66 and the second notch 66 of the manipulating portion 50 is changed to the length L' from the forward end of the sheath 16 to the forward end of the second clip 12B loaded into the sheath 16.

### Embodiment 6

Next, Embodiment 6 of the present invention is described.

While in the embodiments described above the clipping device is placed in the state in which the next clip 12 can be used (standby state) by pulling the sheath 16 to the manipulating portion side, in Embodiment 6, the state in which the next clip 12 can be used is attained by pushing out the manipulating wire 20 to the forward end side.

Here, to be described by way of example is a clipping device in which three clips 12 are loaded for three successive clipping manipulations.

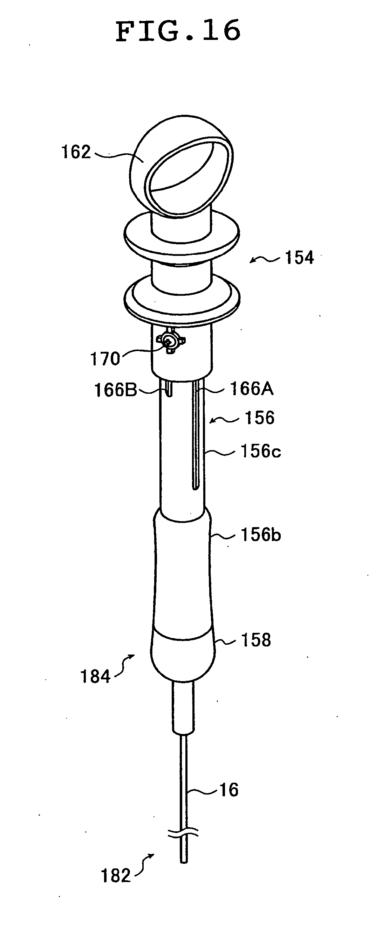

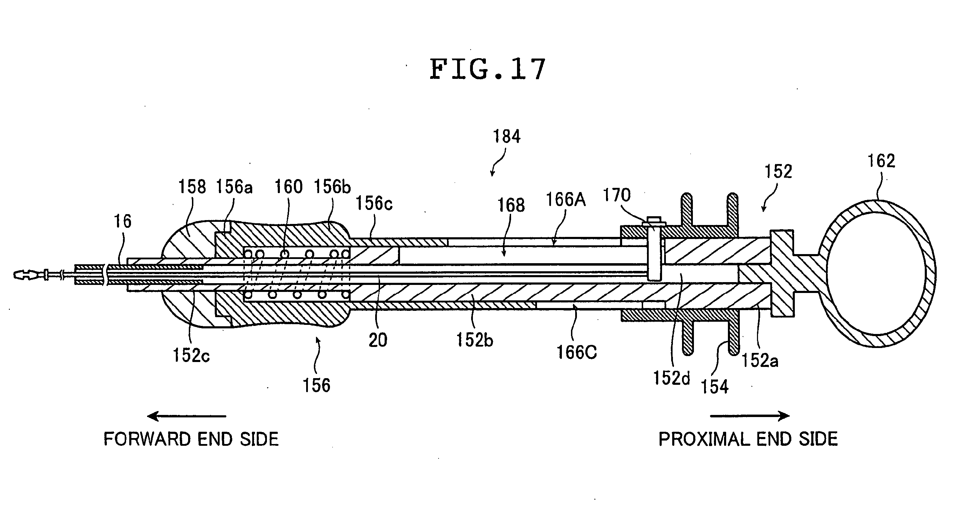

FIGS. 16 and 17 illustrate the construction of a manipulating portion 182 for use in the clipping device of Embodiment 6. The manipulating portion 182 includes the sheath 16, the manipulating wire 20, the connecting member 19 at the forward end of the manipulating wire 20, and a handle portion 184. The handle portion 184 has a handle main body 152, a slider 154, a slider guide 156, a rotating position regulating member 158, an urging spring 160, and a finger hook member 162.

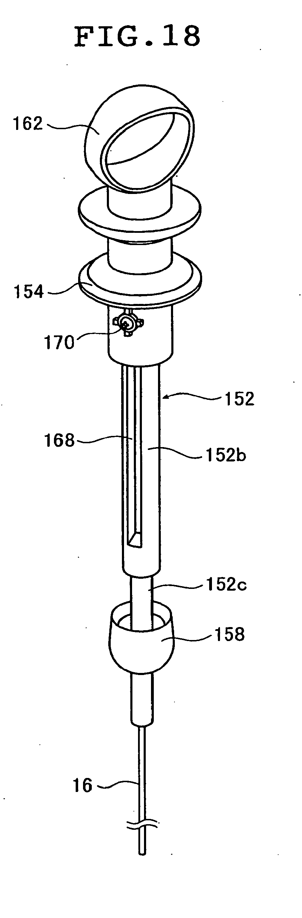

FIG. 18 is a schematic perspective view of the handle main body 152 with the slider guide 156 removed therefrom. The handle main body 152 is a stepped cylindrical member having three cylinder portions differing in outer diameter, and is formed, from the proximal end side, by a large diameter portion 152a, a medium diameter portion 152b, and a small diameter portion 152c.

The handle main body 152 has a through-hole 152d of a fixed diameter extending through the large diameter portion 152a, the medium diameter portion 152b, and the small diameter portion 152c. The finger hook member 162 is fixed to the proximal end side end portion of the large diameter portion 152a by being fixedly fitted into the through-hole 152d. The finger hook member 162 is provided for the doctor to hook his thumb onto it when manipulating the slider 154 described below, and has a ring-like portion.

The medium diameter portion 152b of the handle main body 152 has an engagement groove 168 which is an elongated through-hole extending in the central axis direction of the through-hole 152d. A substantially cylindrical slider guide 156 described below is rotatably inserted into the medium diameter portion 152b.

In the following description, the center axis direction of the cylinder forming the handle main body 152 is referred to as the "axial direction," and the circumferential direction around this axial direction is referred to as the "peripheral direction."

In the handle main body 152, the sheath 16 is fixed to the forward end of the foremost, small diameter portion 152c so as to communicate with the through-hole 152d of the handle main body 152. The manipulating wire 20 is passed through the sheath 16, and protrudes from the proximal end portion of the sheath 16, thereby being passed through the small diameter portion 152c and the medium diameter portion 152b of the handle main body 152 to be connected to the slider 154.

Thus, the sheath 16 is not caused to advance or retreat as in the case of the clipping device 10 illustrated in FIGS. 1A and 1B.

The slider 154 is a substantially cylindrical member which is arranged in the outer periphery of the handle main body 152 so as to pass through the handle main body 152 (and the slider guide 156 described below) and which is movable in the axial direction of the handle main body 152.

The slider 154 has outwardly protruding disc-like flange portions at two positions, that is, the proximal end portion of the cylinder and some midpoint in the axial direction thereof. The operator can hook his finger onto the flange portions and easily move the slider 154 in the axial direction. In an example, the operator inserts his thumb into the ring of the finger hook member 162, and moves the slider 154 in the axial direction while holding the slider 154 between the flange portions between the index finger and the middle finger.

Further, the slider 154 has a slider pin 170 mounted so as to protrude toward the central axis of the handle main body 152. The slider pin 170 passes through the engagement groove 168 to reach the center line of the through-hole 152d of the handle main body 152. Fixed in position in the vicinity of the lower end portion of this slider pin (center line side of the through-hole 152d) is the manipulating wire 20 passed through the smaller diameter portion 152c and the medium diameter portion 152b of the handle main body 152.

As described above, the slider 154 is movable in the axial direction of the handle main body 152. By moving the slider 154, it is possible to cause the manipulating wire 20 inserted into the sheath 16 to advance and retreat (move to the forward end and the proximal end). Through advancement and retreat of the manipulating wire 20 by the slider 154, the clip row at the forward end of the sheath 16 is caused to advance and retreat to place the clipping device in the state in which the next clip 12 can be used.

The position where the proximal end portion of the engagement groove 168 and the slider pin 170 abut each other is the home position (HP) for the slider 154. By moving the slider 154 to the forward end side by a predetermined amount, the manipulating wire 20 is fed toward the forward end side to place the clipping device in the standby state for clipping. By restoring the slider 154 to the HP side from the standby state, the manipulating wire 20 is pulled back, thus effecting clipping and the canceling of the connection between the preceding clip 12 and the succeeding clip 12.

Further, also when loading the clip row into the sheath 16, the slider 154 is moved to the forward end side by a predetermined amount and, in this state, the dummy clip 18 and the manipulating wire 20 are connected together, and the slider 154 is moved to HP, thereby loading the clip row into the sheath 16.

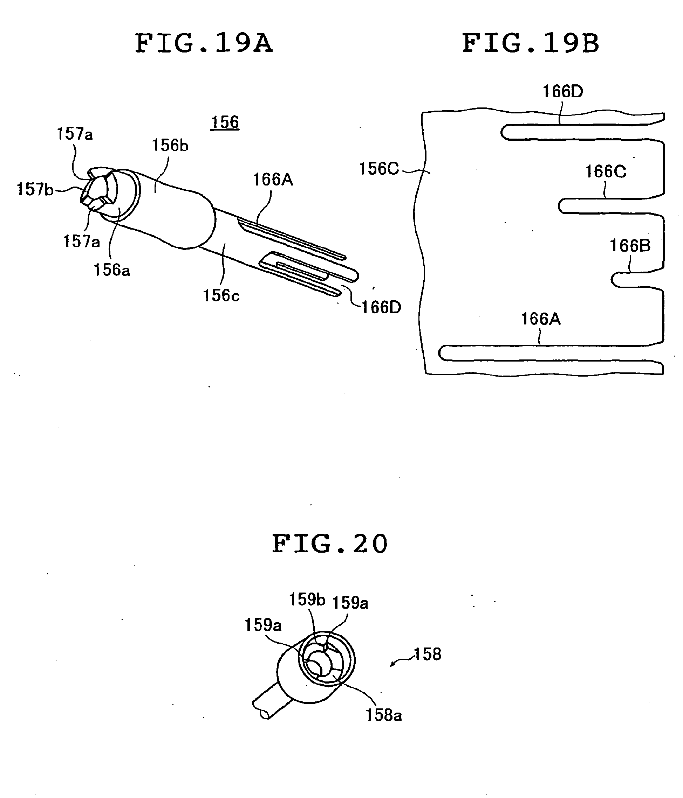

FIG. 19A is a schematic perspective view of a slider guide 156. The slider guide 156 is a substantially cylindrical member for regulating the movement amount in the axial direction of the slider 154, that is, the advancing/retreating amount of the manipulating wire 20 in the longitudinal direction of the sheath 16. The slider guide 156 is supported on the outer peripheral surface of the handle main body 152 so as to be rotatable in the peripheral direction and movable in the axial direction.

The slider guide 156 includes a joint portion 156a, a grasping portion 156b, and a guide portion 156c which are arranged from the forward end side toward the proximal end side and all, of which are substantially cylindrical. The slider guide 156 is formed as an integral unit constituting a single cylinder.

The joint portion 156a has an inner diameter substantially equal to the outer diameter of the smaller diameter portion 152c of the handle main body 152, and its convex forward end portion is inserted into a joint portion 158a formed on a rotating position regulating member 158 for regulating the rotating position of the slider guide 156 described below. The joint portion 156a has four protrusions 157a and four recesses 157b between the protrusions 157a, which are formed in a sawtooth-like fashion. The protrusions 157a and the recesses 157b are engaged with protrusions 159a and recesses 159b formed on the joint portion 158a of the rotating position regulating member 158.

The grasping portion 156b is a portion for grasping to allow the operator to rotate the slider guide 156 to effect clipping as described below.

The guide portion 156c has an inner diameter substantially equal to the outer diameter of the medium diameter portion 152b of the handle main body 152, and an outer diameter substantially equal to the inner diameter of the slider 154 and the outer diameter of the large diameter portion 152a of the handle main body 152. Thus, the slider 154 is guided by the large diameter portion 152a of the handle main body 152 and the outer periphery of the guide portion 156c to move in the axial direction.

FIG. 19B is a developed view of the guide portion 156c. The guide portion 156c has axially extending guide grooves 166A through 166D for guiding the slider 154 (slider pin 170). The guide portion 156c has four guide grooves to conform to a clipping device capable of performing clipping three times with the three clips 12 being loaded and without drawing the sheath 16 out of the living body.

In an example, the guide groove 166A corresponds to the loading of the clip row, the guide groove 166B corresponds to the first clipping, the guide groove 166C corresponds to the second clipping, and the guide groove 166D corresponds to the third clipping, with the guide grooves being formed at a circumferential interval of 90 degrees. In the present invention, the number of clips allowing loading (repeating) is not restricted to three, and the guide portion 156c of the slider guide 156 has (n + 1) guide grooves 166, which corresponds to the number n of clips 12 that can be loaded into the clipping device and one guide groove for clip row loading.

The slider grooves 166A through 166D guide the movement of the slider 154 (slider pin 170) together with the engagement groove 168 of the handle main body 12, and, further, regulate the movement amount of the slider 154. By axially reciprocating the slider 154 from HP, there are conducted clipping manipulation and the loading of the clip row (row formed of three clips 12 and the dummy clip 18 connected together by the connection rings 14) into the sheath 16. Further, it is possible to conduct clipping three times without drawing the sheath 16 out of the living body.

The movement amount of the slider 154 differs according to whether the loading of the clip row is conducted and the number of times that clipping has been conducted. In correspondence with this, as illustrated in FIG. 19B, the slider'guide 156 has four guide grooves 166A through 166D differing in axial length formed in the guide portion 156c. Thus, the lengths of the guide grooves are lengths through which the slider 154 moves at the time of loading of the clip row and in correspondence with the number of times that clipping is performed.

More specifically, at the time of loading of the clip row, it is necessary for the connecting member 19 to protrude from the sheath 16. Further, in the state in which the slider 154 has been restored to HP, it is necessary for the entire region of the clip row to be accommodated in the sheath 16. Thus, as illustrated in FIG. 19B, the guide groove 166A corresponding to the loading of the clip row is formed in a predetermined length which corresponds to maximum movement amount of the slider 154.

Clipping is performed successively starting with the foremost clip 12. As described below, the HP for the clipping manipulation is the same independently of the number of times that clipping is performed. Thus, the requisite movement amount by which the slider moves from HP toward the forward end in order to place the clipping device in the state in which the next clipping is possible, that is, the state in which the arm portions 28 of the clip 12 and the skirt portions 38 of the connection ring 14 protrude from the forward end of the sheath 16, increases gradually as clipping is performed the first, second and third time.

Thus, as illustrated in FIG. 19B, the guide groove 166B corresponding to the first clipping (clip 12A) is formed in a predetermined length leading to the minimum movement amount of the slider 154. Further, the guide groove 166C corresponding to the second clipping (clip 12B) is formed in a predetermined length leading to the second least movement amount of the slider 154. The guide groove 166C corresponding to the third clipping (clip 12C) is formed in a predetermined length leading to the third least movement amount of the slider 154.

The slider guide 156 is rotated according to the manipulation such as the loading of the clip row and clipping, with each guide groove coinciding with the engagement groove 168 of the handle main body 152. That is, the slider guide 156 is rotated such that the guide groove 166A is matched with the engagement groove 168 at the time of loading of the clip row, that the guide groove 166B is matched with the same at the time of the first clipping (clip 12A), that the guide groove 166C is matched with the same at the time of the second clipping (clip 12B), and that the guide groove 166 is matched with the same at the time of the third clipping (clip 12C).

The four protrusions 157a formed at the forward end of the joint portion 156a are of the same configuration, and the four protrusions 157a are of a sawtooth-like configuration, that is, one tooth surface of each of them is gently tapered, and the other tooth surface thereof exhibits a substantially perpendicular step, thus forming a protrusion of a triangular sectional configuration. The intervals between the adjacent protrusions 157a constitute the recesses 157b. The protrusions 157a and the recesses 157b are engaged with the protrusions 159a and the recesses 159b formed on the joint portion 158a of the rotating position regulating member 158.

The rotating position regulating member 158 is a member arranged on the,most proximal side of the handle portion 184, and is a cylindrical member having a cylindrical region and a substantially semi-spherical region, with a through-hole being formed at the center thereof. The rotating position regulating member 158 is fixed to the handle main body 152 by passing the small diameter portion 152c of the handle main body 152 through the through-hole, with the cylindrical region being oriented to the forward end side.

Further, as illustrated in FIG. 20, the rotating position regulating member 158 has a recessed joint portion 158a at the proximal end thereof. As described above, the convex joint portion 156a at the forward end of the slider guide 156 is rotatably inserted into the recessed joint portion 158a.

Like the convex joint portion 156a at the forward end of the slider guide 156, the joint portion 158a has four protrusions 159a of the same configuration which protrude toward the proximal end and which are arranged at equal circumferential intervals, with each of them having two tooth surfaces differing in inclined angle with respect to the abutment surface. The protrusions 159a are formed in a sawtooth-like configuration. That is, one tooth surface of each of them is gently tapered, and the other tooth surface thereof forms a substantially perpendicular, stepped portion, thus forming a protrusion of a triangular sectional configuration. The intervals between the adjacent protrusions 159a are the recesses 159b, which are also four in number.

The protrusions 157a of the joint portion 156a of the slider guide 156 and the recesses 159b of the joint portion 158a of the rotating position regulating member 158 are engaged with each other, and the recesses 157b of the joint portion 156a of the slider guide 156 and the protrusions 159a of the joint portion 158a of the rotating position regulating member 158 are engaged with each other. That is, positioning is effected on the slider guide 156 by the rotating position regulating member 158 at intervals of 90 degrees in the rotating direction.

The guide grooves 166A through 166D of the slider guide 156 are formed such that, when the protrusions and recesses of the joint portion 158a of the rotating position regulating member 158 and the joint portion 156a of the slider guide 156 are engaged with each other, the guide grooves 166A through 166D overlap the engagement grooves 168 of the handle main body 152 in the circumferential direction. That is, the rotation of the slider guide 156 is regulated so as to be stopped by the rotating position regulating member 158 at the position where the guide grooves 166 and the engagement grooves 168 of the handle main body 152 overlap each other.

Each of the protrusions is configured such that one tooth surface has tapered inclined angle and that the other tooth surface is substantially perpendicular, and hence the rotating direction of the slider guide 156 is regulated to one direction. The tooth surfaces of the protrusions are formed such that the guide groove 166A, the guide groove 166B, the guide groove 166C, and the guide groove 166D overlap the engagement groove 168 in that order as the slider guide rotates.

Further, an urging spring 160 is arranged between the step portion between the medium diameter portion 152a and the small diameter portion 152c of the handle main body 152 (i.e., the forward end surface of the medium diameter portion 152b formed by this step portion) and the proximal end surface of the joint portion 156a of the slider guide 156.

The urging spring is a compression spring arranged so as to be wound around the small diameter portion 152c of the handle main body 152. The urging spring exerts an urging force so as to separate the forward end surface of the medium diameter portion 152b and the proximal end surface of the joint portion 156a from each other. That is, the urging spring 160 keeps the slider guide 156 pressed against the rotating position regulating member 158.

Thus, due to the action of the urging spring 160, the slider guide 156 is prevented from being inadvertently rotated.

Further, the slider guide 156 is rotated in a predetermined direction, whereby, due to the protrusions and recesses of the joint portion 158a of the rotating position regulating member 158 and the joint portion 156a of the slider guide 156, the slider guide 156 moves, according to the rotation, toward the proximal end along the tapered portions of the protrusions and recesses of the slider guide 156 against the urging force of the urging spring 160. At the point in time when it is detached from the tapered portions of the protrusions and recesses (the point in time when the protrusions and recesses exhibit substantially perpendicular tooth surfaces), the slider guide 156 moves toward the forward end due to the urging force of the urging spring 160 to be pressed against the rotating position regulating member 158.

As described above, at the position where the protrusions and recesses of the joint portion 158a of the rotating position regulating member 158 and the joint portion 156a of the slider guide 156 are engaged with each other, the engagement groove 168 and the guide grooves 166 are matched with each other in the circumferential direction. Thus, by rotating the slider guide 156, the operator can match the engagement groove 168 with the guide grooves 166 easily and correctly according to the number of times that clipping is performed, etc.

The axial length of the slider guide 156 is set such that, in the state in which it is pressed against the rotating position regulating member 158, there exists, between the step portion between the medium diameter portion 152a and the large diameter portion 152a of the handle main body 152 (i.e., the forward end surface of the large diameter portion 152a formed by this step portion) and the proximal end portion, a gap corresponding to the amount of movement toward the proximal end, etc. due to the protrusions and recesses of the joint portion 158a of the rotating position regulating member 158 and the joint portion 156a of the slider guide 156 at the time of rotation.

With the engagement groove 168 of the handle main body 152 and each guide groove 166 of the slider guide being matched with each other, the slider 154 is moved from HP (position where the proximal end portion of the engagement groove 168 and the slider pin 170 abut each other) to the position where the slider pin abuts the forward end portion of the guide groove 166, and is then returned to HP again, whereby clipping is effected by the clip 12.

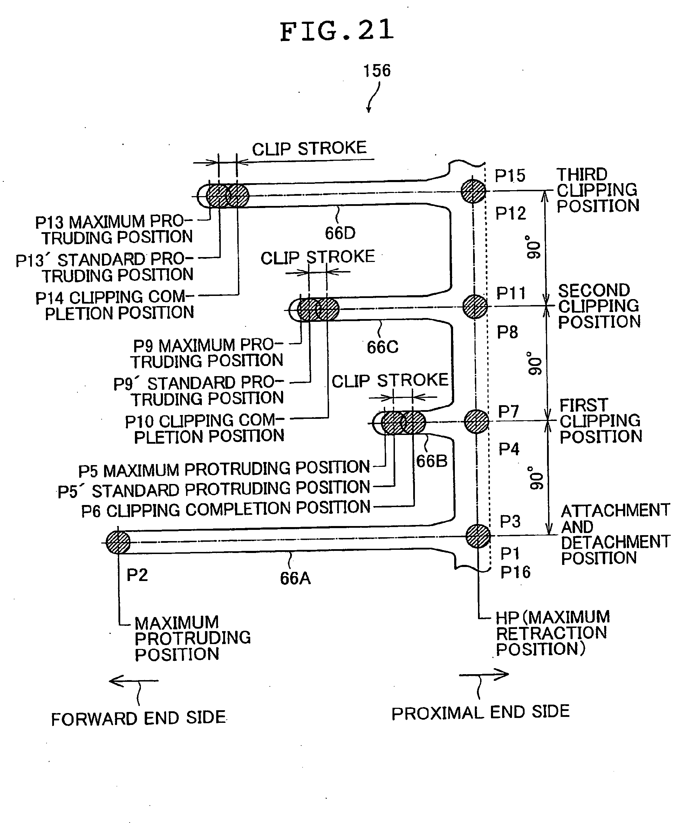

In the following, with reference to FIG. 21, which is a developed view of the slider guide 156, an example of the clipping manipulation conducted three times by the clipping device is described.

First, the slider guide 156 is rotated as needed to match the guide groove 166A with the engagement groove 168 of the handle main body 152, and the slider 154 is moved in the axial direction to HP where the slider pin 170 abuts the forward end surface of the engagement groove 168. That is, the slider pin 170 of the slider 154 is moved to a position P1 illustrated in FIG. 21.

At this time, the forward end of the manipulating wire 20 is retracted into the sheath 16. This state is the initial state of the clipping by the clipping device.

In the present invention, instead of causing the forward end surface of the engagement groove 168 and the slider pin 170 to abut each other, it is also possible to cause the main body of the slider 154 and the forward end surface of the engagement groove 168 to each other, thereby regulating the movement amount in the axial direction of the slider 154.

Next, the slider 154 is moved to the position where it abuts the forward end portion of the guide groove 166A, that is, the slider pin 170 is moved to a maximum protruding position P2. As a result, the forward end of the manipulating wire 20 protrudes by a predetermined amount from the forward end of the sheath 16.

In this state, the connecting member 19 of the dummy clip 18 is attached to the forward end of the manipulating wire 20. As a result, a clip row formed of the three clips 12 and the dummy clip 18 connected together by the connection rings 14 is connected to the manipulating wire 20.

Next, the slider pin 170 is restored to a position P3 illustrated in FIG. 21, that is, to HP. Through this manipulation, the clip row is accommodated in the sheath 16. As a result, the loading of the clip row formed of the clips 12 connected together into the manipulating portion 182 is completed.

After that, the sheath 16 is inserted into the port of the forceps of the endoscope or the like inserted into the living body. Then, the forward end of the sheath 16 is caused to reach the forward end of the insert portion of the endoscope, and is then caused to protrude from the forward end of the endoscope. Further, through manipulation of the insert portion or the angle portion of the endoscope, the forward end of the sheath 16 is moved to the target position.

When the requisite manipulation has been completed, the slider guide 156 is rotated by 90 degrees to match the guide groove 166B with the engagement groove 168. As a result, the position of the slider pin 170 is moved to a position P4 in FIG. 21, that is, HP, which corresponds to the guide groove 166B.

Next, the slider 154 is moved to the position where it abuts the forward end portion of the guide groove 166B, that is, to a maximum protruding position P5 in FIG. 21. Through this extrusion of the slider 154, that is, the extrusion of the manipulating wire 20, the clip row is moved in the direction of the forward end, and the foremost clip 12A and the first region 32 of the connection ring 14A protrude from the forward end of the sheath 16. As a result, the arm portions 28 of the clip 12A are opened, and further, the skirt portions 38 of the connection ring 14A are opened.

It should be noted that there is dimensional variation or the like due to a production error in the clips 12 and the connection rings 14. Further, in the clipping device inserted into the endoscope, there may be a case in which the protruding amount of the manipulating wire 20 decreases due to a difference between the inner and outer periphery, etc. attributable to bending, curving, etc. of the manipulating wire 20 and the sheath 16. Thus, the forward end of the guide groove 166B is at the maximum protruding position P5 where the clip 12A is not detached.from the sheath 16, and where the skirt portions 38 of the connection ring 14A are reliably opened independently of a production error in the clips 12, etc. or the condition of the sheath 16.

Thus, normally, in the state in which the slider pin 170 has been pushed forward to the maximum protruding position P5, the skirt portions 38 of the connection ring 14A are situated in front of the forward end portion of the sheath 16, and the skirt portions 38 and the sheath 16 are spaced apart from each other.

This also applies to the forward end portion of the guide groove 166C corresponding to the second clipping by the clip 12B, and to the forward end portion of the guide groove 166D corresponding to the third clipping by the clip 12C.

Next, while watching, for example, the display of the endoscope, the operator restores the slider pin 170 to the HP side, and restores the clip row to the sheath 16 to the standard protruding position P5' where the skirt portions 38 of the connection ring 14A abut the forward end portion of the sheath 16. As a result, the preparation for the first clipping (clipping by the first clip 12) is completed.

After that, the endoscope is operated to press the claw portions 22 of the diverged clip 12A against the portion of the living body to be subjected to clipping, and, in this state, the slider pin 170 is moved to the proximal side to be restored to HP, that is, the position P7.

Through this movement of the slider pin 170, the foremost clip 12A is drawn into the connection ring 14A, and the arm portions 28, which have been open, are closed by the clamping ring 40, with the claw portions 22 being closed to effect clipping on the living body. When the slider pin 170 moves from the standard protruding position P5' to the clipping completion position P6, the portions of the arm portions 28 directly below the projections 30 are drawn into the connection ring 14A, whereby the clipping is completed.

Simultaneously with the completion of the clipping, the proximal end portion of the foremost clip 12A (proximal end portion of turned portion 24) and the claw portions 22 of the second clip 12B are discharged from the proximal end portion of the connection ring 14A. As a result, the arm portions 28 of the second clip 12B, which have been closed by the second region 34 of the connection ring 19A, are opened up to the inner diameter of the sheath 16, and the engagement between the turned portion 24 of the preceding clip 12A and the claw portions of the next clip 12B is released, whereby the clip 12A and the connection ring 14A are separated from the clip row, thereby attaining the state in which the clip 12A and the connection ring 14A can be discharge from the sheath 16.

Further, in the state in which the slider pin 170 has been restored to the position P7, the clip row separated from the clip12A and the connection ring 14A is drawn into the sheath 16.

As is apparent from the above description, the distance between the maximum protruding position P5 (P9, P13) and the standard protruding position P5' (P9', P13') serves as a buffer for absorbing a production error in the components, a difference between the inner and outer periphery of the sheath 16, etc. Thus, by once pushing out the slider pin 170 to the maximum protruding position P5, it is possible to reliably open the arm portions 28 and the skirt portions 38 to perform clipping independently of the production error in the clips 12 or the condition of the sheath 16 in the living body.

In a preferable manipulation, the slider pin 170 is pushed out to the maximum protruding position P5, and then returned to the standard protruding position P5'. After that, the claw portions 22 are brought into contact with the living body to effect clipping (restoration of the slider 54 to P7, which is HP), whereby it is possible to more reliably prevent detachment, etc. of the clip 12 attributable to excessive protrusion from the sheath 16. Further, it is possible to press the reliably retained clip 12 firmly against the living body to be subjected to clipping.

When, at the maximum protruding position, the foremost clip 12 is firmly retained, and there is no (or very little) risk of detachment, the slider pin 170 may be pulled back at a stroke from the maximum protruding position to HP to effect clipping and the releasing of the connection of the clip row.

It is also desirable to generate a small impact (i.e., so-called click feel) by well-known means such as a protrusion and a recess engaged with each other or an urged spherical body and a recess engaged therewith at the point in time when the slider pin 170 passes the clipping completion position P6 (P10, P14), thus enabling the operator performing the clipping to be aware of the completion of the clipping.

When the slider pin 170 has been restored to the position P7, which is HP, to complete the first clipping (clipping by the first clip 12A), the slider guide 156 is rotated by 90 degrees as illustrated in FIG. 12 (H) to match the guide groove 166C with the engagement groove 168. As a result, the position of the slider pin 170 moves to HP, which corresponds to the guide groove 166C, as indicated at P8 in FIG. 21.

Next, the slider pin 170 is moved to the maximum protruding position P9 where the slider pin 170 abuts the forward end portion of the guide groove 166C. Through this manipulation, the second clip 12B and the first region 32 of the connection ring 14B protrude from the forward end of the sheath 16, with the arm portions 28 and the skirt portions 38 opening. Further, by pulling the slider pin 170 back to the standard protruding position P9' where the skirt portions 38 abut the forward end of the sheath 16, the clipping device is placed in the state in which the clipping device is ready for the second clipping (by the clip 12B).

When the clipping device has become ready for clipping, the claw portions 22 of the diverged clip 12B are pressed against the portion which is to be subjected to clipping, and the slider pin 170 is moved to the proximal side to be pulled back to HP, that is, the position P11.

As a result, through the movement of the slider pin 170 from the standard protruding position P9' to the clipping completion position P10, the clipping by the second clip 12 is completed, and the second clip 12B and the next clip 12C (one on the'most proximal side) are separated from each other, whereby a state is attained in which the clip 12B and the connection ring 14B can be discharged from the sheath 16.

In the state in which the slider pin 170 has been restored to the position P11, which is HP, the clip row separated from the clip 12B and the connection ring 14B is in the state in which the clip row has been drawn into the sheath 16.

When the second clipping is completed, the slider guide 156 is then rotated by 90 degrees to match the guide groove 166D with the engagement groove 168. As a result, the position of the slider pin 170 moves to HP, which corresponds to the guide groove 166D, indicated as the position P12 in FIG. 21.

Next, the slider pin 170 is moved to the maximum protruding position P13 where the slider pin 170 abuts the forward end portion of the guide groove 166D. Through this manipulation, the third clip 12C and the connection ring 14B protrude from the forward end of the sheath 16, with the arm portions 28 and the skirt portions 38 opening. Further, by pulling the slider pin 170 back to the standard protruding position P13', the clipping device is placed in the state in which the clipping device is ready for the third clipping.

When the clipping device has become ready for clipping, the claw portions 22 of the diverged clip 12C are pressed against the portion which is to be subjected to clipping, and the slider pin 170 is moved to the proximal side to be pulled back to HP, that is, the position P15.

As a result, clipping is performed in the same manner as described above, and the clipping by the third clip 12C is completed through the movement of the slider pin 170 from the standard protruding position P13' to the clipping completion position P14, and, further, the third clip 12C and the dummy clip 18 are separated from each other, whereby the state is attained in which the clip 12C and the connection ring 14C can be discharged from the sheath 16.

In the state in which the slider pin 170 has been restored to the position P15, which is HP, the dummy clip 18 separated from all the clips is in the state in which the dummy clip 18 has been drawn into the sheath 16.

When the clipping by the three clips 12 has been completed, the slider guide 156 is rotated by 90 degrees to match the guide groove 166A with the engagement groove 168. As a result, the position of the slider pin 170 is restored again to HP, which corresponds to the guide groove 166A as indicated by the position P1 in FIG. 21. After that, the sheath 16 is pulled out of the endoscope.

After the sheath 16 has been pulled out, the slider pin 170 is pushed out to the position P2 where the slider pin 170 abuts the forward end portion of the guide groove 166A, and the dummy clip 18 and the connecting member 19 are caused to protrude from the forward end of the sheath 16, thereby removing the dummy clip 18 and the connecting member 19 from the forward end of the manipulating wire 20.

As described above, it is possible to perform clipping a plurality of times without pulling out the sheath. Further, solely through the rotation of the slider guide 156 and the reciprocating movement of the slider 154, the clip row is moved in the axial direction (longitudinal direction of the sheath 16) by a proper amount according to the number of times that clipping is performed (first time, second time ...) to place the clipping device in the state in which the clipping device is ready for clipping, making it possible to perform clipping and the separation of the clips connected together. That is, it is possible to perform accurate clipping through easy manipulation.

The clipping device of the embodiments of the present invention described in detail above should not be construed restrictively. It goes without saying that various improvements and variations are possible without departing from the scope of the present claims. The clipping device of the present invention is applicable not only to a soft endoscope but also to a hard endoscope.

## Claims

1. A clipping device comprising:
a plurality of clips (12A, 12B ...) loaded into a forward end portion of a sheath (16) while being engaged with other clips longitudinally connected together, each clip having arm portions (28);
a manipulating wire (20) connected to a rear end of the rearmost clip (12E) and used to pull a clip row formed of the plurality of clips (12);
at least one connection ring (14, 114) fitted into the sheath (16) so as to be capable of advancing and retreating, the connection ring (14, 114) covering an engagement portion of the clips (12) to maintain the clips (12) in a connected state;
wherein the connection ring (14, 114) includes:
a resin retaining portion (42, 142) having a first region (32) for retaining the clip (12A) and a second region (34) for retaining the connection of the next clip (12B) to the clip (12A), wherein the resin retaining portion (42) has, at the same position in a clip pulling direction and at positions circumferentially spaced apart from each other, two ore more skirt portions (38) which, when being situated inside the sheath (16), are closed inwardly by being pressed by an inner wall of the sheath (16) and which, when being situated outside of a forward end of the sheath (16), are opened in a width larger than an inner diameter of the sheath to prevent retreat into the sheath (16), and
a metal clamping portion (40) arranged on a forward end side of the retaining portion (42,142) and, when being situated on a forward end side of the clip (12), abutting the clip to clamp the clip (12), **characterized in that**
two arm portion accommodating grooves (43a) opposed to each other are formed within the second region (34) to guide the connection of the arm portions of the next clip (12B) to the rear end of the clip (12A).

2. The clipping device according to claim 1,
wherein the plurality of clips (12A; 12B ...) are connected together with orientations thereof being changed alternately by 90 degrees.

3. The clipping device according to claim 1, wherein when the skirt portions (38) of the connection ring (14) are inwardly closed inside the sheath (16), at least one of a pair of clips (12) connected together in the connection ring (14) is retained through pressurization.

4. The clipping device according to any one of claims 1 through 3, wherein the clip (12A) is pulled with respect to the connection ring (14A) corresponding by the manipulating wire (20), and an engagement portion between the clip (12A), and the next clip (12B) is detached from the connection ring (14A) to thereby release the clip (12A) from the connection with the next clip (12B).

5. The clipping device according to any one of Claims 1 through 4, wherein, after the clip (12A) has been released from the connection and used for clipping, the sheath (16) is pulled down to a position where next clip (12B) protrudes, whereby the next clip becomes usable.

## Patentansprüche

1. Klammervorrichtung umfassend:
Eine Mehrzahl von Klammern (12A, 12B ...), die in den vorderen Endabschnitt einer Hülle (16) geladen sind, während sie mit anderen Klammern in Längsrichtung verbunden sind und jede Klammer Armabschnitte (28) aufweist;
Einen Handhabungsdraht (20), der an ein hinteres Ende der hintersten Klammer (12E) angeschlossen ist und dazu dient, eine aus den mehreren Klammern (12) gebildete Klammerreihe zu ziehen;
mindestens einen Verbindungsring (14, 114), der in die Hülle (16) derart eingepasst ist, dass er vorgerückt und zurückgezogen werden kann, wobei der Verbindungsring (14, 114) einen Eingriffsbereich der Klammern (12) bedeckt, um die Klammern (12) in einem verbundenen Zustand zu halten;
wobei der Verbindungsring (14, 114) beinhaltet:
einen Kunststoff-Haltebereich (42, 142) mit einer ersten Zone (32) zum Halten der Klammer (12A) und einer zweiten Zone (34) zum Halten der Verbindung der nächsten Klammer (B) zu der Klammer (12A), wobei der Kunststoff-Haltebereich (42) an der gleichen Stelle in Klammer-Ziehrichtung und an voneinander über den Umfang beabstandeten Stellen zwei oder mehr Schürzenabschnitte (38) aufweist, die, wenn sie sich innerhalb der Hülle (16) befinden, nach innen geschlossen sind und von einer Innenwand der Hülle (16) unter Druck gesetzt werden, und die, wenn sie sich außerhalb eines vorderen Endes der Hülle (16) befinden, in einer Breite geöffnet werden, die größer ist als ein Innendurchmesser der Hülle, um ein Zurückziehen in die Hülle (16) zu verhindern, und
einen Metall-Klemmabschnitt (14), der an einer vorderen Endseite des Haltebereichs (42, 142) angeordnet ist und, wenn er sich an einer vorderen Endseite der klammer (12) befindet, an der Klammer anliegt, um die Klammer (12) zu klemmen, **dadurch gekennzeichnet, dass**
zwei Armabschnitt-Aufnahmenuten (43a) einander gegenüber liegend innerhalb der zweiten Zone (34) ausgebildet sind, um die Verbindung der am Abschnitt der nächsten Klammer (12B) zu dem hinteren Ende der Klammer (12A) zu führen.

2. Klammervorrichtung nach Anspruch 1,
bei der die mehreren Klammern (12A; 12B) miteinander verbunden sind, indem ihre Orientierungen abwechselnd um 90° wechseln.

3. Klammervorrichtung nach Anspruch 1, bei der, wenn die Schürzenabschnitte (38) des Verbindungsrings (14) im Inneren der Hülle (16) nach innen geschlossen sind, mindestens eine Klammer von einem in dem Verbindungsring (14) miteinander verbundenem Paar von Klammern durch Druckbeaufschlagung gehalten wird.

4. Klammervorrichtung nach einem der Ansprüche 1 bis 3, bei der die Klammer (12A) bezüglich des entsprechenden Verbindungsrings (14A) von dem Handhabungsdraht (3) gezogen wird und ein Eingriffsabschnitt zwischen der Klammer (12A) und der nächsten Klammer (12B) von dem Verbindungsring (14A) gelöst wird, um **dadurch** die Klammer (12A) aus der Verbindung mit der nächsten Klammer (12B) zu lösen.

5. Klammervorrichtung nach einem der Ansprüche 1 bis 4, bei der, nachdem die Klammer (12A) aus der Verbindung gelöst und zum Klammern eingesetzt wurde, die Hülle (16) zu einer Stelle nach unten gezogen wird, an der die nächste Klammer (12B) vorsteht, wodurch die nächste Klammer verwendbar wird.

## Revendications

1. Dispositif d'agrafage comprenant :
une pluralité d'agrafes (12A, 12B...) chargées dans une partie d'extrémité avant d'une gaine (16) tout en étant mises en prise avec d'autres agrafes longitudinalement raccordées ensemble, chaque agrafe ayant des parties de bras (28) ;
un fil de manipulation (20) raccordé à une extrémité arrière de l'agrafe située le plus en arrière (12E) et utilisé pour tirer une rangée d'agrafes formée par la pluralité d'agrafes (12) ;
au moins un anneau de raccordement (14, 114) monté dans la gaine (16) afin de pouvoir avancer et reculer, l'anneau de raccordement (14, 114) couvrant une partie de mise en prise des agrafes (12) pour maintenir les agrafes (12) dans un état raccordé ;
dans lequel l'anneau de raccordement (14, 114) comprend :
une partie de retenue en résine (42, 142) ayant une première région (32) pour retenir l'agrafe (12A) et une deuxième région (34) pour retenir le raccordement de l'agrafe suivante (12B) par rapport à l'agrafe (12A), dans lequel la partie de retenue de résine (42) a, à la même position, dans une direction de traction d'agrafe et dans des positions circonférentiellement espacées les unes des autres, deux parties de jupe ou plus (38) qui, lorsqu'elles sont situées à l'intérieur de la gaine (16), sont fermées vers l'intérieur en étant comprimées par une paroi interne de la gaine (16) et qui, lorsqu'elles sont situées vers l'extérieur d'une extrémité avant de la gaine (16), sont ouvertes dans une largeur supérieure à un diamètre interne de la gaine afin d'empêcher le recul dans la gaine (16), et
une partie de serrage métallique (40) agencée sur un côté d'extrémité avant de la partie de retenue (42, 142) et, lorsqu'elle est située sur un côté d'extrémité avant de l'agrafe (12), venant en butée contre l'agrafe pour serrer l'agrafe (12), **caractérisé en ce que** :
deux rainures de logement de partie de bras (43a) opposées entre elles sont formées à l'intérieur de la deuxième région (34) pour guider le raccordement des parties de bras de l'agrafe suivante (12B) sur l'extrémité arrière de l'agrafe (12A).

2. Dispositif d'agrafage selon la revendication 1, dans lequel la pluralité d'agrafes (12A ; 12B...) sont raccordées ensemble avec leurs orientations qui sont modifiées de manière alternée de 90 degrés.

3. Dispositif d'agrafage selon la revendication 1, dans lequel lorsque les parties de jupe (38) de l'anneau de raccordement (14) sont fermées vers l'intérieur, à l'intérieur de la gaine (16), au moins l'une d'une paire d'agrafes (12) raccordées conjointement avec l'anneau de raccordement (14), est retenue par le biais de la mise sous pression.

4. Dispositif d'agrafage selon l'une quelconque des revendications 1 à 3, dans lequel l'agrafe (12A) est tirée par rapport à l'anneau de raccordement (14A) de manière correspondante par le fil de manipulation (20), et une partie de mise en prise entre l'agrafe (12A) et l'agrafe suivante (12B) est détachée de la bague de raccordement (14A) pour libérer ainsi l'agrafe (12A) du raccordement avec l'agrafe suivante (12B).

5. Dispositif d'agrafage selon l'une quelconque des revendications 1 à 4, dans lequel, après que l'agrafe (12A) a été libérée du raccordement et utilisée pour l'agrafage, la gaine (16) est retirée dans une position dans laquelle l'agrafe suivante (12B) fait saillie, moyennant quoi l'agrafe suivante peut être utilisée.
